# EUROPEAN PATENT APPLICATION

(11) **EP 4 553 078 A1**
(43) Date of publication of application: **14.05.2025**
(21) Application number: 23835509.3
(22) Date of filing: 04.07.2023
(51) Int. Cl.: C07D 487/04, A61K 31/519, A61P 35/00

(54) **5H-PYRROLO[2,3-D]PYRIMIDIN-6(7H)-ONE AND CRYSTAL OF SALT THEREOF**

(30) Priority: 05.07.2022 JP 2022108654
(71) Applicant: Taiho Pharmaceutical Co., Ltd., Chiyoda-ku, Tokyo 101-8444 (JP)
(72) Inventor: EGASHIRA, Naoki, Tsukuba-shi, Ibaraki 300-2611 (JP); HASHIMOTO, Masaya, Tsukuba-shi, Ibaraki 300-2611 (JP); OSHIUMI, Hiromi, Kodama-gun, Saitama 367-0241 (JP); SUGIMOTO, Tetsuya, Tsukuba-shi, Ibaraki 300-2611 (JP); KOBAYAKAWA, Yu, Tsukuba-shi, Ibaraki 300-2611 (JP); YAMAMOTO, Fuyuki, Tsukuba-shi, Ibaraki 300-2611 (JP)
(74) Representative: Wächtershäuser & Hartz Patentanwaltspartnerschaft mbB
(86) International application number: PCT/JP2023/024703
(87) International publication number: WO 2024/009977

(57) **Abstract**

Provided is a type I crystal of a free base of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which has peaks at, at least, 2 diffraction angles (2θ±0.2°) selected from the following (a), and at, at least, 2 diffraction angles (2θ±0.2°) selected from the following (b), in a powder X-ray diffraction spectrum (CuKα):
(a) 6.2°, 9.3°, 9.7°, 11.1°, 15.4° and 25.1°; and
(b) 6.7°, 8.3°, 8.7°, 13.0°, 13.7°, 16.3°, 16.9°, 17.7°, 18.7°, 19.4°, 20.3°, 25.9° and 26.5°.

## Description

### Technical Field

The present disclosure relates to: crystals of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one and a saltthereof, which are excellent in terms of preservation stability and hygroscopicity and are useful as pharmaceutical products or drug substances for pharmaceutical products; and pharmaceutical compositions comprising said crystals.

### Background Art

In general, when a compound is used as an active ingredient of a pharmaceutical product, the compound needs to be chemically and physically stable in order to stably maintain its quality and/or to facilitate storage and management.

As a compound that has excellent inhibitory activity against at least one selected from the group consisting of Akt, Rsk and S6K, and/or has an action to suppress cancer cell proliferation, and/or is useful as a medical care for treating various diseases (in particular, cancer) involving at least one selected from the group consisting of Akt, Rsk and S6K, Patent Literature 1 discloses 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one (hereinafter also referred to as "Compound (I)"), which is represented by the following formula (I):

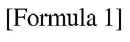

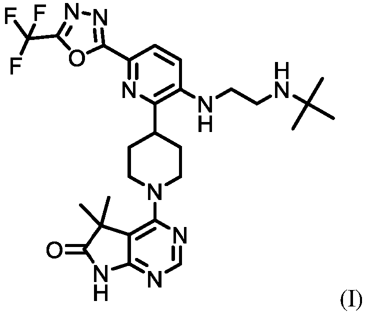

However, Patent Literature 1 does not disclose a crystal form of the present compound, and the stable crystal form of Compound (I) and a method for producing the same are not currently known.

### Citation List

### Patent Literature

Patent Literature 1: International Publication WO2017/200087

### Summary of Invention

### Technical Problem

It is an object of the present disclosure to provide a crystal of a free base of Compound (I) and a crystal of a salt thereof, which are useful as pharmaceutical products or drug substances for pharmaceutical products.

### Solution to Problem

As a result of intensive studies directed towards achieving the aforementioned object, the present inventors have found three crystal forms (type I crystal, type II crystal, and type III crystal) as free bodies of Compound (I). Furthermore, the present inventors have discovered a crystal of a hydrochloride of Compound (I) and a crystal of a maleate of Compound (I). The present inventors have found that these crystals have excellent solid state stability and low hygroscopicity.

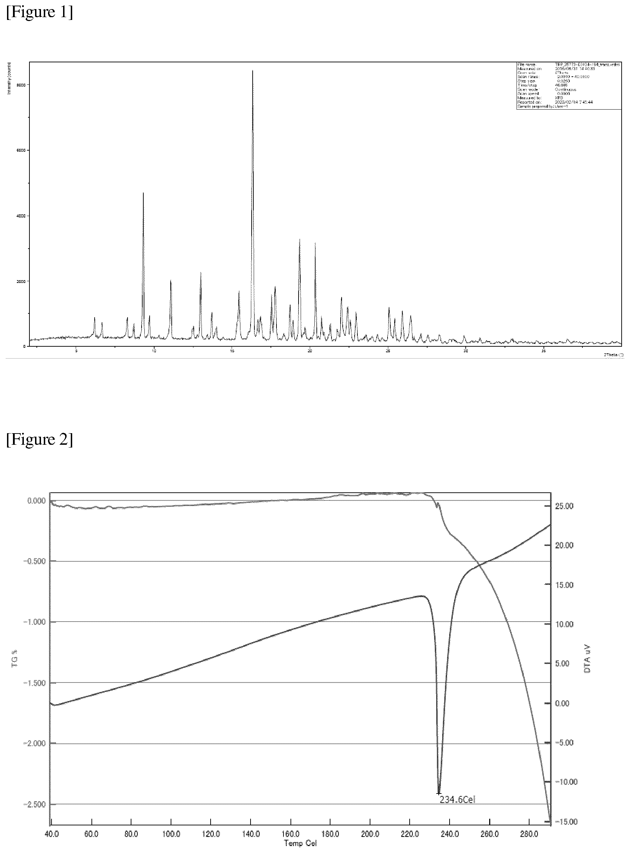

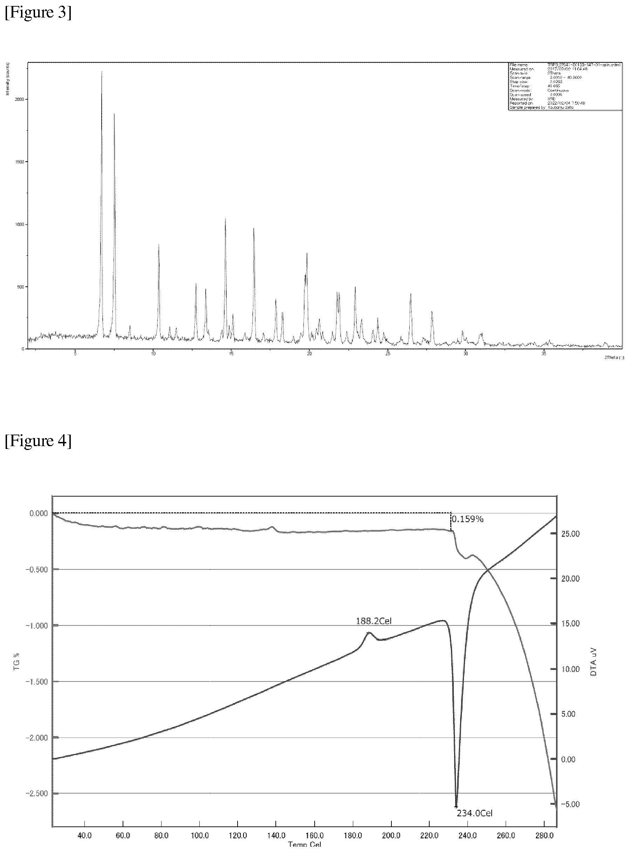

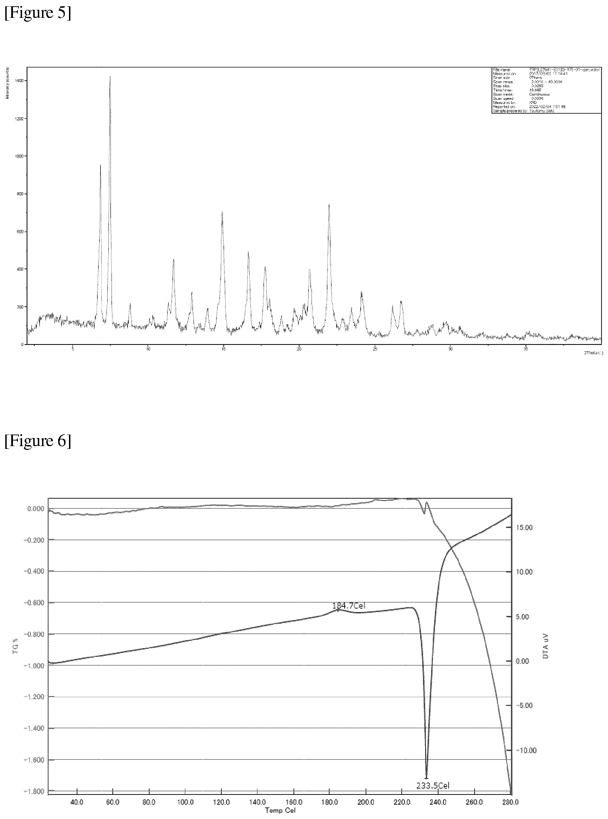

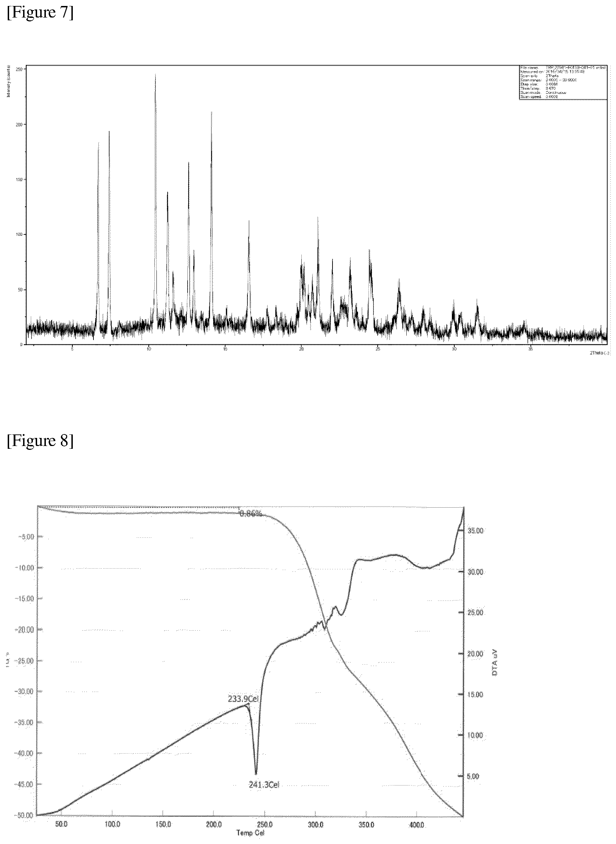

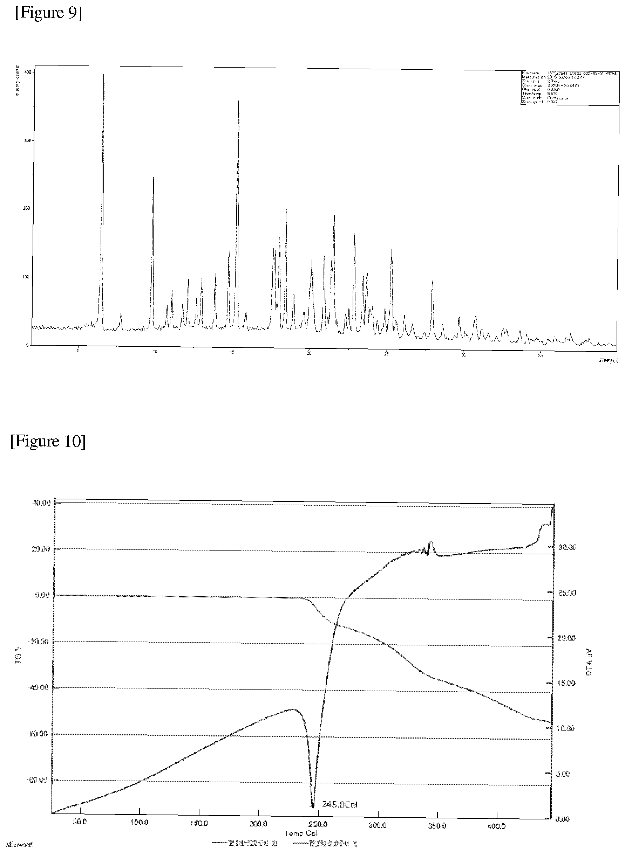

Specifically, the present disclosure provides the following [1] to [32].
[1] A type I crystal of a free base of 4-(4-(3-((2-(tert-butylamino)ethyl)amino)-6-(5-(trifluoromethyl)-1,3,4-oxadiazol-2-yl)pyridin-2-yl)piperidin- 1-yl)-5,5-dimethyl-5H-pyrrolo[2,3-d]pyrimidin-6(7H)-one, which has peaks at, at least, 2 diffraction angles (2θ±0.2°) selected from the following (a), and at, at least, 2 diffraction angles (2θ±0.2°) selected from the following (b), in a powder X-ray diffraction spectrum (CuKα):
   (a) 6.2°, 9.3°, 9.7°, 11.1°, 15.4° and 25.1°; and
   (b) 6.7°, 8.3°, 8.7°, 13.0°, 13.7°, 16.3°, 16.9°, 17.7°, 18.7°, 19.4°, 20.3°, 25.9° and 26.5°.
[2] The type I crystal of the free base according to the above [1], which has peaks at, at least, 3 diffraction angles (2θ±0.2°) selected from the following (a), and at, at least, 4 diffraction angles (2θ±0.2°) selected from the following (b), in a powder X-ray diffraction spectrum (CuKα):
   (a) 6.2°, 9.3°, 9.7°, 11.1°, 15.4° and 25.1°; and
   (b) 6.7°, 8.3°, 8.7°, 13.0°, 13.7°, 16.3°, 16.9°, 17.7°, 18.7°, 19.4°, 20.3°, 25.9° and 26.5°.
[3] The type I crystal of the free base according to the above [1], which has peaks at the following diffraction angles (2θ±0.2°): 6.2°, 6.7°, 8.3°, 8.7°, 9.3°, 9.7°, 11.1°, 13.0°, 13.7°, 15.4°, 16.3°, 16.9°, 17.7°, 18.7°, 19.4°, 20.3°, 25.1°, 25.9°, and 26.5°, in a powder X-ray diffraction spectrum (CuKα).
[4] The type I crystal of the free base according to the above [1], which is a crystal having a powder X-ray diffraction spectrum substantially identical to the powder X-ray diffraction spectrum shown in Figure 1.
[5] The type I crystal of the free base according to the above [1], wherein the peak temperature in thermogravimetry-differential thermal analysis (TG-DTA) has an endothermic peak of around 235°C.
[6] A pharmaceutical composition, comprising the type I crystal of the free base according to any one of the above [1] to [5].
[7] A method for producing the type I crystal of the free base according to the above [1], comprising a step of dissolving 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one in a solvent 1, and a step of adding a solvent 2 to the resulting solution to obtain the 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one.
[8] The method according to the above [7], wherein the solvent 1 is dimethyl sulfoxide, and the solvent 2 is water, or the solvent 1 is tetrahydrofuran and the solvent 2 is normal heptane, ethyl acetate, or 2-propanol.
[9] The method according to the above [7], wherein the solvent 1 is dimethyl sulfoxide and the solvent 2 is water.
[10] The type I crystal of the free base according to the above [1], which is obtained via crystallization by performing a step of dissolving 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one in dimethyl sulfoxide, and a step of adding water to the resulting solution to obtain the 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one.
[11] A type II crystal of a free base of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which has peaks at, at least, 2 diffraction angles (2θ±0.2°) selected from the following (a), and at, at least, 2 diffraction angles (2θ±0.2°) selected from the following (b), in a powder X-ray diffraction spectrum (CuKα):
   (a) 10.4°, 22.9° and 27.9°; and
   (b) 6.7°, 7.5°, 12.7°, 13.4°, 14.6°, 16.5°, 17.9°, 18.3°, 19.8°, 20.6°, 21.8° and 26.5°.
[12] The type II crystal of the free base according to the above [11], which has peaks at, at least, 3 diffraction angles (2θ±0.2°) selected from the following (a), and at, at least, 4 diffraction angles (2θ±0.2°) selected from the following (b), in a powder X-ray diffraction spectrum (CuKα):
   (a) 10.4°, 22.9° and 27.9°; and
   (b) 6.7°, 7.5°, 12.7°, 13.4°, 14.6°, 16.5°, 17.9°, 18.3°, 19.8°, 20.6°, 21.8° and 26.5°.
[13] The type II crystal of the free base according to the above [11], which has the following diffraction angles (2θ±0.2°): 6.7°, 7.5°, 10.4°, 12.7°, 13.4°, 14.6°, 16.5°, 17.9°, 18.3°, 19.8°, 20.6°, 21.8°, 22.9°, 26.5° and 27.9°, in a powder X-ray diffraction spectrum (CuKα).
[14] The type II crystal of the free base according to the above [11], which is a crystal having a powder X-ray diffraction spectrum substantially identical to the powder X-ray diffraction spectrum shown in Figure 3.
[15] The type II crystal of the free base according to the above [11], wherein the peak temperature in thermogravimetry-differential thermal analysis (TG-DTA) has an endothermic peak of around 234°C.
[16] A pharmaceutical composition, comprising the type II crystal of the free base according to any one of the above [11] to [15].
[17] A type III crystal of a free base of 4-(4-(3-((2-(tert-butylamino)ethyl)amino)-6-(5-(trifluoromethyl)-1,3,4-oxadiazol-2-yl)pyridin-2-yl)piperidin- 1-yl)-5,5-dimethyl-5H-pyrrolo[2,3-d]pyrimidin-6(7H)-one, which has peaks at, at least, 2 diffraction angles (2θ±0.2°) selected from the following (a), and at, at least, 2 diffraction angles (2θ±0.2°) selected from the following (b), in a powder X-ray diffraction spectrum (CuKα):
   (a) 11.7°, 23.4° and 24.2°; and
   (b) 6.8°, 7.5°, 8.8°, 12.9°, 13.9°, 14.9°, 16.7°, 17.8°, 18.8°, 19.7°, 20.7°, 22.0°, 26.2° and 26.7°.
[18] The type III crystal of the free base according to the above [17], which has peaks at, at least, 3 diffraction angles (2θ±0.2°), and at, at least, 4 diffraction angles (2θ±0.2°) selected from the following (b), in a powder X-ray diffraction spectrum (CuKα):
   (a) 11.7°, 23.4° and 24.2°; and
   (b) 6.8°, 7.5°, 8.8°, 12.9°, 13.9°, 14.9°, 16.7°, 17.8°, 18.8°, 19.7°, 20.7°, 22.0°, 26.2° and 26.7°.
[19] The type III crystal of the free base according to the above [17], which has peaks at the following diffraction angles (2θ±0.2°): 6.8°, 7.5°, 8.8°, 11.7°, 12.9°, 13.9°, 14.9°, 16.7°, 17.8°, 18.8°, 19.7°, 20.7°, 22.0°, 23.4°, 24.2°, 26.2° and 26.7°, in a powder X-ray diffraction spectrum (CuKα).
[20] The type III crystal of the free base according to the above [17], which is a crystal having a powder X-ray diffraction spectrum substantially identical to the powder X-ray diffraction spectrum shown in Figure 5.
[21] The type III crystal of the free base according to the above [17], wherein the peak temperature in thermogravimetry-differential thermal analysis (TG-DTA) has an endothermic peak of around 234°C.
[22] A pharmaceutical composition, comprising the type III crystal of the free base according to any one of the above [17] to [21].
[23] A crystal of a hydrochloride of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which has peaks at, at least, 3 diffraction angles selected from the following diffraction angles (2θ±0.2°): 6.7°, 7.4°, 11.2°, 12.6°, 14.1°, 16.6°, 20.0°, 21.1°, 22.0°, 22.6°, 23.2°, 24.5°, 26.4°, 28.0°, 30.0° and 31.6°, in a powder X-ray diffraction spectrum (CuKα).
[24] The crystal of the hydrochloride according to the above [23], which has peaks at the following diffraction angles (2θ±0.2°): 6.7°, 7.4°, 11.2', 12.6°, 14.1°, 16.6°, 20.0°, 21.1°, 22.0°, 22.6°, 23.2°, 24.5°, 26.4°, 28.0°, 30.0° and 31.6°, in a powder X-ray diffraction spectrum (CuKα).
[25] The crystal of the hydrochloride according to the above [23], which is a crystal having a powder X-ray diffraction spectrum substantially identical to the powder X-ray diffraction spectrum shown in Figure 7.
[26] The crystal of the hydrochloride according to the above [23], wherein the peak temperature in thermogravimetry-differential thermal analysis (TG-DTA) has an endothermic peak of around 241°C.
[27] A pharmaceutical composition, comprising the crystal of the hydrochloride according to any one of the above [23] to [26].
[28] A crystal of a maleate of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which has peaks at, at least, 3 diffraction angles selected from the following diffraction angles (2θ±0.2°): 6.5°, 9.8°, 12.1°, 13.0°, 13.9°, 14.7°, 15.2°, 15.9°, 17.7°, 18.4°, 19.0°, 20.1°, 20.9°, 21.5°, 22.9°, 23.4°, 25.3°, 28.0°, 29.7° and 30.8°, in a powder X-ray diffraction spectrum (CuKα).
[29] The crystal of the maleate according to the above [28], which has peaks at the following diffraction angles (2θ±0.2°): 6.5°, 9.8°, 12.1°, 13.0°, 13.9°, 14.7°, 15.2°, 15.9°, 17.7°, 18.4°, 19.0°, 20.1°, 20.9°, 21.5°, 22.9°, 23.4°, 25.3°, 28.0°, 29.7° and 30.8°, in a powder X-ray diffraction spectrum (CuKα).
[30] The crystal of the maleate according to the above [28], which is a crystal having a powder X-ray diffraction spectrum substantially identical to the powder X-ray diffraction spectrum shown in Figure 9.
[31] The crystal of the maleate according to the above [28], wherein the peak temperature in thermogravimetry-differential thermal analysis (TG-DTA) has an endothermic peak of around 245°C.
[32] A pharmaceutical composition, comprising the crystal of the maleate according to any one of the above [28] to [31].

Furthermore, the present disclosure provides the following [33] to [60].
[33] A method for treating tumor, comprising a step of administering the pharmaceutical composition according to the above [6] to a subject.
[34] A method for treating tumor, comprising a step of administering the pharmaceutical composition according to the above [16] to a subject.
[35] A method for treating tumor, comprising a step of administering the pharmaceutical composition according to the above [22] to a subject.
[36] A method for treating tumor, comprising a step of administering the pharmaceutical composition according to the above [27] to a subject.
[37] A method for treating tumor, comprising a step of administering the pharmaceutical composition according to the above [32] to a subject.
[38] A type I crystal of a free base of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which is for use in the treatment of tumor, wherein
   the crystal has peaks at, at least, 2 diffraction angles (2θ±0.2°) selected from the following (a), and at, at least, 2 diffraction angles (2θ±0.2°) selected from the following (b), in a powder X-ray diffraction spectrum (CuKα):
   (a) 6.2°, 9.3°, 9.7°, 11.1°, 15.4° and 25.1°; and
   (b) 6.7°, 8.3°, 8.7°, 13.0°, 13.7°, 16.3°, 16.9°, 17.7°, 18.7°, 19.4°, 20.3°, 25.9° and 26.5°.
[39] The type I crystal of the free base according to the above [38], which has peaks at, at least, 3 diffraction angles (2θ±0.2°) selected from the following (a), and at, at least, 4 diffraction angles (2θ±0.2°) selected from the following (b), in a powder X-ray diffraction spectrum (CuKα):
   (a) 6.2°, 9.3°, 9.7°, 11.1°, 15.4° and 25.1°; and
   (b) 6.7°, 8.3°, 8.7°, 13.0°, 13.7°, 16.3°, 16.9°, 17.7°, 18.7°, 19.4°, 20.3°, 25.9° and 26.5°.
[40] The type I crystal of the free base according to the above [38], which has peaks at the following diffraction angles (2θ±0.2°): 6.2°, 6.7°, 8.3°, 8.7°, 9.3°, 9.7°, 11.1°, 13.0°, 13.7°, 15.4°, 16.3°, 16.9°, 17.7°, 18.7°, 19.4°, 20.3°, 25.1°, 25.9° and 26.5°, in a powder X-ray diffraction spectrum (CuKα).
[41] The type I crystal of the free base according to the above [38], which is a crystal having a powder X-ray diffraction spectrum substantially identical to the powder X-ray diffraction spectrum shown in Figure 1.
[42] The type I crystal of the free base according to the above [38], wherein the peak temperature in thermogravimetry-differential thermal analysis (TG-DTA) has an endothermic peak of around 235°C.
[43] A type II crystal of a free base of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which is for use in the treatment of tumor, wherein
   the crystal has peaks at, at least, 2 diffraction angles (2θ±0.2°) selected from the following (a), and at, at least, 2 diffraction angles (2θ±0.2°) selected from the following (b), in a powder X-ray diffraction spectrum (CuKα):
   (a) 10.4°, 22.9° and 27.9°; and
   (b) 6.7°, 7.5°, 12.7°, 13.4°, 14.6°, 16.5°, 17.9°, 18.3°, 19.8°, 20.6°, 21.8° and 26.5°.
[44] The type II crystal of the free base according to the above [43], which has peaks at, at least, 4 diffraction angles (2θ±0.2°) selected from the following (b):
   (a) 10.4°, 22.9° and 27.9°; and
   (b) 6.7°, 7.5°, 12.7°, 13.4°, 14.6°, 16.5°, 17.9°, 18.3°, 19.8°, 20.6°, 21.8° and 26.5°.
[45] The type II crystal of the free base according to the above [43], which has peaks at the following diffraction angles (2θ±0.2°): 6.7°, 7.5°, 10.4°, 12.7°, 13.4°, 14.6°, 16.5°, 17.9°, 18.3°, 19.8°, 20.6°, 21.8°, 22.9°, 26.5° and 27.9°, in a powder X-ray diffraction spectrum (CuKα).
[46] The type II crystal of the free base according to the above [43], which is a crystal having a powder X-ray diffraction spectrum substantially identical to the powder X-ray diffraction spectrum shown in Figure 3.
[47] The type II crystal of the free base according to the above [43], wherein the peak temperature in thermogravimetry-differential thermal analysis (TG-DTA) has an endothermic peak of around 234°C.
[48] A type III crystal of a free base of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which is for use in the treatment of tumor, wherein
   the crystal has peaks at, at least, 2 diffraction angles (2θ±0.2°) selected from the following (a), and at, at least, 2 diffraction angles (2θ±0.2°) selected from the following (b), in a powder X-ray diffraction spectrum (CuKα):
   (a) 11.7°, 23.4° and 24.2°; and
   (b) 6.8°, 7.5°, 8.8°, 12.9°, 13.9°, 14.9°, 16.7°, 17.8°, 18.8°, 19.7°, 20.7°, 22.0°, 26.2° and 26.7°.
[49] The type III crystal of the free base according to the above [48], which has peaks at, at least, 4 diffraction angles (2θ±0.2°) selected from the following (b):
   (a) 11.7°, 23.4° and 24.2°; and
   (b) 6.8°, 7.5°, 8.8°, 12.9°, 13.9°, 14.9°, 16.7°, 17.8°, 18.8°, 19.7°, 20.7°, 22.0°, 26.2° and 26.7°.
[50] The type III crystal of the free base according to the above [48], which has peaks at the following diffraction angles (2θ±0.2°): 6.8°, 7.5°, 8.8°, 11.7°, 12.9°, 13.9°, 14.9°, 16.7°, 17.8°, 18.8°, 19.7°, 20.7°, 22.0°, 23.4°, 24.2°, 26.2° and 26.7°, in a powder X-ray diffraction spectrum (CuKα).
[51] The type III crystal of the free base according to the above [48], which is a crystal having a powder X-ray diffraction spectrum substantially identical to the powder X-ray diffraction spectrum (CuKα) shown in Figure 5.
[52] The type III crystal of the free base according to the above [48], wherein the peak temperature in thermogravimetry-differential thermal analysis (TG-DTA) has an endothermic peak of around 234°C.
[53] A crystal of a hydrochloride of a free base of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which is for use in the treatment of tumor, wherein
   the crystal has peaks at, at least, 3 diffraction angles selected from the following diffraction angles (2θ±0.2°): 6.7°, 7.4°, 11.2°, 12.6°, 14.1°, 16.6°, 20.0°, 21.1°, 22.0°, 22.6°, 23.2°, 24.5°, 26.4°, 28.0°, 30.0° and 31.6°, in a powder X-ray diffraction spectrum (CuKα).
[54] The crystal of the hydrochloride according to the above [52], which has peaks at the following diffraction angles (2θ±0.2°): 6.7°, 7.4°, 11.2', 12.6°, 14.1°, 16.6°, 20.0°, 21.1°, 22.0°, 22.6°, 23.2°, 24.5°, 26.4°, 28.0°, 30.0° and 31.6°, in a powder X-ray diffraction spectrum (CuKα).
[55] The crystal of the hydrochloride according to the above [53], which is a crystal having a powder X-ray diffraction spectrum substantially identical to the powder X-ray diffraction spectrum (CuKα) shown in Figure 7.
[56] The crystal of the hydrochloride according to the above [53], wherein the peak temperature in thermogravimetry-differential thermal analysis (TG-DTA) has an endothermic peak of around 241°C.
[57] A crystal of a maleate of a free base of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which is for use in the treatment of tumor, wherein
   the crystal has peaks at, at least, 3 diffraction angles selected from the following diffraction angles (2θ±0.2°): 6.5°, 9.8°, 12.1°, 13.0°, 13.9°, 14.7°, 15.2°, 15.9°, 17.7°, 18.4°, 19.0°, 20.1°, 20.9°, 21.5°, 22.9°, 23.4°, 25.3°, 28.0°, 29.7° and 30.8°, in a powder X-ray diffraction spectrum (CuKα).
[58] The crystal of the maleate according to the above [57], which has peaks at the following diffraction angles (2θ±0.2°): 6.5°, 9.8°, 12.1°, 13.0°, 13.9°, 14.7°, 15.2°, 15.9°, 17.7°, 18.4°, 19.0°, 20.1°, 20.9°, 21.5°, 22.9°, 23.4°, 25.3°, 28.0°, 29.7° and 30.8°, in a powder X-ray diffraction spectrum (CuKα).
[59] The crystal of the maleate according to the above [57], which is a crystal having a powder X-ray diffraction spectrum substantially identical to the powder X-ray diffraction spectrum (CuKα) shown in Figure 9.
[60] The crystal of the maleate according to the above [57], wherein the peak temperature in thermogravimetry-differential thermal analysis (TG-DTA) has an endothermic peak of around 245°C.

### Advantageous Effects of Invention

According to the present disclosure, there is provided a stable crystal form of the above-described Compound (I), which is useful as a compound having an antitumor action. Moreover, the stable crystal form of Compound (I) of the present disclosure and a method for producing the same are not currently known. In the present disclosure, crystal forms of a free base of Compound (I) (i.e., type I crystal, type II crystal, and type III crystal), a crystal of a hydrochloride of Compound (I), and a crystal of a maleate of Compound (I) are superior to other crystal forms, for example, from the viewpoint of low hygroscopicity and/or solid state stability, and thus, these are useful forms, when the present compound is used as a drug substance for pharmaceutical products.

### Brief Description of Drawings

[Figure 1] Figure 1 shows the powder X-ray diffraction spectrum (CuKα) of the type I crystal of the free base of Compound (I) obtained in Example 1 (the vertical axis represents intensity (counts), and the horizontal axis represents diffraction angle (2θ)).
[Figure 2] Figure 2 shows the thermogravimetry-differential thermal analysis (TG-DTA) curve of the type I crystal of the free base of Compound (I) obtained in Example 1.
[Figure 3] Figure 3 shows the powder X-ray diffraction spectrum (CuKα) of the type II crystal of the free base of Compound (I) obtained in Example 2 (the vertical axis represents intensity (counts), and the horizontal axis represents diffraction angle (2θ)).
[Figure 4] Figure 4 shows the thermogravimetry-differential thermal analysis (TG-DTA) curve of the type II crystal of the free base of Compound (I) obtained in Example 2.
[Figure 5] Figure 5 shows the powder X-ray diffraction spectrum (CuKα) of the type III crystal of the free base of Compound (I) obtained in Example 3 (the vertical axis represents intensity (counts), and the horizontal axis represents diffraction angle (2θ)).
[Figure 6] Figure 6 shows the thermogravimetry-differential thermal analysis (TG-DTA) curve of the type III crystal of the free base of Compound (I) obtained in Example 3.
[Figure 7] Figure 7 shows the powder X-ray diffraction spectrum (CuKα) of the crystal of the hydrochloride of Compound (I) obtained in Example 4 (the vertical axis represents intensity (counts), and the horizontal axis represents diffraction angle (2θ)).
[Figure 8] Figure 8 shows the thermogravimetry-differential thermal analysis (TG-DTA) curve of the crystal of the hydrochloride of Compound (I) obtained in Example 4.
[Figure 9] Figure 9 shows the powder X-ray diffraction spectrum (CuKα) of the crystal of the maleate of Compound (I) obtained in Example 5 (the vertical axis represents intensity (counts), and the horizontal axis represents diffraction angle (2θ)).
[Figure 10] Figure 10 shows the thermogravimetry-differential thermal analysis (TG-DTA) curve of the crystal of the maleate of Compound (I) obtained in Example 5.
[Figure 11] Figure 11 shows the powder X-ray diffraction spectrum (CuKα) of the crystal of the 1/2 malate of Compound (I) obtained in Comparative Example 1 (the vertical axis represents intensity (counts), and the horizontal axis represents diffraction angle (2θ)).
[Figure 12] Figure 12 shows the thermogravimetry-differential thermal analysis (TG-DTA) curve of the crystal of the 1/2 malate of Compound (I) obtained in Comparative Example 1.
[Figure 13] Figure 13 shows the powder X-ray diffraction spectrum (CuKα) of the crystal of the acetate of Compound (I) obtained in Comparative Example 2 (the vertical axis represents intensity (counts), and the horizontal axis represents diffraction angle (2θ)).
[Figure 14] Figure 14 shows the thermogravimetry-differential thermal analysis (TG-DTA) curve of the crystal of the acetate of Compound (I) obtained in Comparative Example 2.
[Figure 15] Figure 15 shows the moisture adsorption/desorption isotherm of the type I crystal of the free base of Compound (I) obtained in Example 1.
[Figure 16] Figure 16 shows the moisture adsorption/desorption isotherm of the type II crystal of the free base of Compound (I) obtained in Example 2.
[Figure 17] Figure 17 shows the moisture adsorption/desorption isotherm of the type III crystal of the free base of Compound (I) obtained in Example 3.
[Figure 18] Figure 18 shows the powder X-ray diffraction data of the type I crystal of the free base of Compound (I) obtained in Example 1 at 25°C.
[Figure 19] Figure 19 shows the powder X-ray diffraction data of the type I crystal of the free base of Compound (I) obtained in Example 1 at 210°C.
[Figure 20] Figure 20 shows the powder X-ray diffraction data of the type II crystal of the free base of Compound (I) obtained in Example 2 at 25°C.
[Figure 21] Figure 21 shows the powder X-ray diffraction data of the type II crystal of the free base of Compound (I) obtained in Example 2 at 210°C.
[Figure 22] Figure 22 shows the powder X-ray diffraction data of the type III crystal of the free base of Compound (I) obtained in Example 3 at 25°C.
[Figure 23] Figure 23 shows the powder X-ray diffraction data of the type III crystal of the free base of Compound (I) obtained in Example 3 at 210°C.
[Figure 24] Figure 24 shows the moisture adsorption/desorption isotherm of the crystal of the hydrochloride of Compound (I) obtained in Example 4.
[Figure 25] Figure 25 shows the moisture adsorption/desorption isotherm of the crystal of the maleate of Compound (I) obtained in Example 5.
[Figure 26] Figure 26 shows the moisture adsorption/desorption isotherm of the crystal of the 1/2 malate of Compound (I) obtained in Comparative Example 1.
[Figure 27] Figure 27 shows the moisture adsorption/desorption isotherm of the crystal of the acetate of Compound (I) obtained in Comparative Example 2.

### Description of Embodiments

In the present description, when merely the term "Compound (I)" is used, it means 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, and this term is used to mean to include both an "amorphous form" and a "crystal."

In the salt of Compound (I), the molar equivalent of the acid relative to Compound (I) can be analyzed, for example, by NMR or liquid chromatography.

In the present disclosure, when the phrase "a crystal of the hydrochloride, maleate or acetate of Compound (I)" is used, the molar ratio between Compound (I) and hydrochloric acid, maleic acid or acetic acid is 1 : 1, and can be determined by analysis such as 1H-NMR. In general, in a salt of an organic compound, the molar ratio between the acid and the organic compound can be determined by comparing the integral value of the methyl proton of the acid with the integral value of at least one proton of the organic compound. Since it is known that an error occurs in the integral value of the proton due to correction of the baseline of the 1H-NMR chart, etc., when the molar ratio between the acid and the organic compound is in the range of 0.8 to 1.2 : 1, it can be considered to be 1 : 1.

Compound (I) can be synthesized, for example, according to the method described in International Publication WO2017/200087 (for example, the method described in Example 32 of the present publication), but the synthetic method is not limited thereto.

When the following terms are used in the present description, unless otherwise clearly specified, if the term "crystal" and related terms used in the present description are used to describe a compound, a substance, modification, a material, an ingredient, or a product, these terms mean that the compound, substance, modification, material, ingredient, or product is substantially a crystal when determined by X-ray diffraction. Please refer to, for example, Remington: The Science and Practice of Pharmacy, 21st edition, Lippincott, Williams and Wilkins, Baltimore, MD (2005); United States Pharmacopeia, 23rd edition, 1843-1844 (1995). The term "crystal" also include a hydrate or a solvate (pseudopolymorph), and a cocrystal. The terms "crystal" and "crystalline form" are used interchangeably in the present description.

In the present technical field, even in a multi-component system capable of forming salts (accompanied by the transfer of protons due to acid-base reactions), it is known that a main component (a drug substance for pharmaceutical products) and subcomponents (a coformer, a counterion, and a solvent) may form a complex through a weak intermolecular interaction and the like, such as a hydrogen bond (e.g., Cryst. Growth Des. 2012, 12, 2147-2152 dx.doi.org/10.1021/cg3002948).

The "crystal" of a "salt" in the present disclosure also includes such a crystal of a complex formed by multiple components (i.e., a "cocrystal").

As used in the present description, and unless otherwise clearly specified, the terms "amorphous" and "amorphous form," and related terms used in the present description mean that the substance, component, or product is not substantially a crystal when determined by X-ray diffraction. In particular, the term "amorphous form" describes an irregular solid form, i.e., a solid form that lacks a crystalline order over a long distance.

The term " pharmaceutically acceptable salt" refers to a salt derived from a variety of organic and inorganic counterions known in the present technical field. The pharmaceutically acceptable salt may be safe for ingestion by animals or humans.

The techniques of determining the properties of a crystalline form and an amorphous form that can be used herein may include, but are not limited to, publicly known techniques in the present technical field, such as thermogravimetric analysis (TGA), differential scanning calorimetry (DSC), X-ray powder diffraction (XRPD), single-crystal X-ray diffraction, vibrational spectroscopy such as, for example, infrared (IR) and Raman spectroscopy, solid-state nuclear magnetic resonance (NMR) spectroscopy, optical microscopy, hot stage optical microscopy, scanning electron microscopy (SEM), electron crystallography, quantitative analysis, and solubility measurement. Characteristic unit cell parameters can be determined using one or more techniques, such as, but not limited to, X-ray diffraction and neutron diffraction, including single crystal diffraction and powder diffraction as typical examples. The technique useful for analyzing powder diffraction data may be profile refinement such as Rietveld refinement, which can be used, for example, to analyze diffraction peaks associated with a single phase in a sample containing two or more solid phases. Another method useful for analyzing powder diffraction data may be unit cell indexing, and this method allows those skilled in the art to determine unit cell parameters from samples containing crystalline powders.

It is to be noted that, due to the properties of the data of the powder X-ray diffraction patterns, the diffraction angle and overall pattern are important when determining the identity of the crystal. Since the relative intensity of a powder X-ray diffraction pattern may vary somewhat depending on the direction of crystal growth, the size of the particles, and the measurement conditions, it should not be interpreted strictly.

The crystal is specified by a peak in a powder X-ray diffraction pattern. Herein, the "peak" is selected to be one having 5% or more, 6% or more, 7% or more, 8% or more, 9% or more, 10% or more, 15% or more, or 20% or more of the intensity of the maximum peak among the intensity peaks obtained by the powder X-ray diffraction spectrum (CuKα). More preferably, the peak is 5% or more of the intensity of the maximum peak among the obtained intensity peaks, more preferably 6% or more of the intensity of the maximum peak among the obtained intensity peaks, even more preferably 7% or more of the intensity of the maximum peak among the obtained intensity peaks, further preferably 8% or more of the intensity of the maximum peak among the obtained intensity peaks, still further preferably 9% or more of the intensity of the maximum peak among the obtained intensity peaks, still further preferably 10% or more of the intensity of the maximum peak among the obtained intensity peaks, still further preferably 15% or more of the intensity of the maximum peak among the obtained intensity peaks, and particularly preferably 20% or more of the intensity of the maximum peak among the obtained intensity peaks.

The numerical values obtained from various patterns may have some errors depending on the direction of crystal growth, the size of the particles, the measurement conditions, etc. Thus, in the present description, the numerical value of the diffraction angle (2θ) in the powder X-ray diffraction pattern may have a measurement error of about ±0.2°.

In addition, with regard to the endothermic peak in the thermogravimetry-differential thermal analysis (TG-DTA) curve, the measurement temperature may vary, depending on the temperature rise width per minute, the purity of the sample, etc. In the present description, the term "around" in the TG-DTA measured temperature value means a temperature value within ±2, 3, 4, or 5°C of the value. The temperature range is preferably ±5°C, more preferably ±4°C, even more preferably ±3°C, and particularly preferably ±2°C.

In a certain embodiment , the crystalline form of Compound (I) disclosed in the present description may contain multiple crystals (crystal polymorphs) having spatially regular atomic arrangements and may result in different physicochemical properties. In a certain embodiment , the crystalline form disclosed in the present description may be a mixture containing crystal polymorphs.

The crystalline form of Compound (I) of the present disclosure may be characterized by a combination of the below-mentioned embodiments, which do not contradict one another. For example, the crystalline form of Compound (I) may be characterized by a combination of the above-described peaks in an X-ray diffraction pattern and the above-described endothermic peaks measured by TG-DTA.

Compounds labeled with isotopes (e.g., deuterium, 3H, 14C, 35S, 125I, etc.), etc. are also included in the compound described in the present description or a pharmaceutically acceptable salt thereof.

The crystalline forms of some Compounds (I) disclosed in the present description are stable, when they are exposed to conditions of a temperature of about 25°C, 30°C, 35°C, 40°C, 45°C, 50°C, 55°C, 60°C, 65°C or 70°C, and a relative humidity of about 65%, 70%, 75%, 80% or 85%, for about 1, 2, 3, 4, 5, 6, 7, 8, 12, 16, 24, 36, 48, 60, 72, 84, 96, 108, 120, 132, 144, or 156 weeks or more, and for about 160, 156, 150, 138, 126, 114, 102, 90, 78, 66, 54, 42, 30, 24, 18, 12, or 6 weeks or less. The conditions may be closed conditions or open conditions. As used in the present description, the "closed" conditions mean conditions in which the lids of the bottles containing samples are closed or hermetically sealed during stability experiments, whereas the "open" conditions mean conditions in which the lids of the bottles are open.

The crystalline form of Compound (I) disclosed in the present description is stable upon exposure to conditions of about 70°C and a relative humidity of about 75% for about 2 weeks. That is, the crystalline form of Compound (I) disclosed in the present description exhibits excellent storage stability over a long period of time. In the present description, "stable" means that the increase in impurities is about 1.0%, 0.5%, 0.4%, 0.3%, 0.2%, 0.1%, 0.05%, or 0.01% or less, compared to the initial amount of impurities. The increase in impurities is preferably 1.0% or less, more preferably 0.5% or less, even more preferably 0.4% or less, further preferably 0.3% or less, still further preferably 0.2% or less, still further preferably 0.1% or less, still further preferably 0.05% or less, and particularly preferably 0.01% or less.

In a certain embodiment, the crystalline form of Compound (I) disclosed in the present description can be physically and/or chemically highly pure. In specific embodiments, the crystal disclosed in the present description can be, at least, about 100%, 99%, 98%, 97%, 96%, 95%, 94%, 93%, 92%, 91%, 90%, 89%, 88%, 87%, 86%, 85%, 84%, 83%, 82%, 81%, or 80% physically and/or chemically pure. In some embodiments, the crystal disclosed in the present description is substantially pure. As used in the present description, and unless otherwise clearly specified, the term "substantially pure" means that, for example, the present crystal does not substantially contain other solid forms and/or other compounds. In specific embodiments, the crystal disclosed in the present description contains about 20%, 15%, 10%, 9%, 8%, 7%, 6%, 5%, 4%, 3%, 2%, 1%, 0.75%, 0.5%, 0.25%, or less than 0.1% by weight of one or more other solid forms and/or other compounds.

The type I crystal of the free base of Compound (I) has, for example, the powder X-ray diffraction spectrum (CuKα) shown in Figure 1 and the thermogravimetry-differential thermal analysis (TG-DTA) curve shown in Figure 2.

Herein, the characteristic peaks of the type I crystal of the free base of Compound (I) in the powder X-ray diffraction spectrum may be, as diffraction angles (2θ±0.2°), 6.2°, 6.7°, 8.3°, 8.7°, 9.3°, 9.7°, 11.1°, 13.0°, 13.7°, 15.4°, 16.3°, 16.9°, 17.7°, 18.7°, 19.4°, 20.3°, 25.1°, 25.9°, and 26.5°. More preferably, the characteristic peaks of the type I crystal of the free base of Compound (I) in the powder X-ray diffraction spectrum (CuKα) have diffraction angles (2θ±0.2°) of 6.2°, 6.7°, 8.3°, 8.7°, 9.3°, 9.7°, 11.1°, 13.0°, 13.7°, 15.4°, 16.3°, 16.9°, 17.7°, 18.7°, 19.4°, 20.3°, 25.1°, 25.9° and 26.5°, when measured by the reflection method.

The type I crystal of the free base of Compound (I) in one embodiment of the present disclosure has peaks at, at least, 2 diffraction angles (2θ±0.2°) selected from the following (a), and at, at least, 2 diffraction angles (2θ±0.2°) selected from the following (b), in the powder X-ray diffraction spectrum (CuKα):
(a) 6.2°, 9.3°, 9.7°, 11.1°, 15.4° and 25.1°; and
(b) 6.7°, 8.3°, 8.7°, 13.0°, 13.7°, 16.3°, 16.9°, 17.7°, 18.7°, 19.4°, 20.3°, 25.9° and 26.5°.

The type I crystal of the free base of Compound (I) in a preferred embodiment of the present disclosure has, as diffraction angles (2θ±0.2°), at least 2 peaks selected from the above-described group (a), and at least 2 peaks selected from the above-described group (b), in the powder X-ray diffraction spectrum. More preferably, the type I crystal of the free base of Compound (I) has, as diffraction angles (2θ±0.2°), at least 3 peaks selected from the above-described group (a), and at least 2 peaks selected from the above-described group (b), in the powder X-ray diffraction spectrum. Even more preferably, the type I crystal of the free base of Compound (I) has, as diffraction angles (2θ±0.2°), at least 4 peaks selected from the above-described group (a), and at least 2 peaks selected from the above-described group (b), in the powder X-ray diffraction spectrum. Further preferably, the type I crystal of the free base of Compound (I) has, as diffraction angles (2θ±0.2°), at least 5 peaks selected from the above-described group (a), and at least 2 peaks selected from the above-described group (b), in the powder X-ray diffraction spectrum. Particularly preferably, the type I crystal of the free base of Compound (I) has, as diffraction angles (2θ±0.2°), all peaks selected from the above-described group (a), and at least 2 peaks selected from the above-described group (b), in the powder X-ray diffraction spectrum.

In another embodiment, the type I crystal of the free base of Compound (I) in a preferred embodiment of the present disclosure has, as diffraction angles (2θ±0.2°), at least 2 peaks selected from the above-described group (a), and at least 2, preferably at least 3, more preferably at least 4, even more preferably at least 5, further preferably at least 6, still further preferably at least 7, still further preferably at least 8, still further preferably at least 9, still further preferably at least 10, still further preferably at least 11, still further preferably at least 12, and particularly preferably all peaks selected from the above-described group (b), in the powder X-ray diffraction spectrum.

In another embodiment, the type I crystal of the free base of Compound (I) has, as diffraction angles (2θ±0.2°), at least 3 peaks selected from the above-described group (a), and at least 2, preferably at least 3, more preferably at least 4, even more preferably at least 5, further preferably at least 6, still further preferably at least 7, still further preferably at least 8, still further preferably at least 9, still further preferably at least 10, still further preferably at least 11, still further preferably at least 12, and particularly preferably all peaks selected from the above-described group (b), in the powder X-ray diffraction spectrum.

In another embodiment, the type I crystal of the free base of Compound (I) has, as diffraction angles (2θ±0.2°), at least 4 peaks selected from the above-described group (a), and at least 2, preferably at least 3, more preferably at least 4, even more preferably at least 5, further preferably at least 6, still further preferably at least 7, still further preferably at least 8, still further preferably at least 9, still further preferably at least 10, still further preferably at least 11, still further preferably at least 12, and particularly preferably all peaks selected from the above-described group (b), in the powder X-ray diffraction spectrum.

In another embodiment, the type I crystal of the free base of Compound (I) has, as diffraction angles (2θ±0.2°), at least 5 peaks selected from the above-described group (a), and at least 2, preferably at least 3, more preferably at least 4, even more preferably at least 5, further preferably at least 6, still further preferably at least 7, still further preferably at least 8, still further preferably at least 9, still further preferably at least 10, still further preferably at least 11, still further preferably at least 12, and particularly preferably all peaks selected from the above-described group (b), in the powder X-ray diffraction spectrum.

In another embodiment, the type I crystal of the free base of Compound (I) has, as diffraction angles (2θ±0.2°), all peaks selected from the above-described group (a), and at least 2, preferably at least 3, more preferably at least 4, even more preferably at least 5, further preferably at least 6, still further preferably at least 7, still further preferably at least 8, still further preferably at least 9, still further preferably at least 10, still further preferably at least 11, still further preferably at least 12, and particularly preferably all peaks selected from the above-described group (b), in the powder X-ray diffraction spectrum.

In addition, the endothermic peak of the type I crystal of the free base of Compound (I) in the thermogravimetry-differential thermal analysis (TG-DTA) curve may be around 233°C to 237°C, and preferably around 235°C.

In the type I crystal of the free base of Compound (I), the chemical purity of Compound (I) is 90% or more, and it can be measured by high performance liquid chromatography (HPLC). It is preferably the type I crystal of the free base of Compound (I) in which the chemical purity of Compound (I) is 95% or more, and it is more preferably the type I crystal of the free base of Compound (I) in which the chemical purity of Compound (I) is 99% or more.

The type I crystal of the free base of Compound (I) may be any crystal, as long as it contains a free base type I crystal, and it may be either a single crystal of type I crystal of a free base, or a polymorphic mixture containing crystals other than the type I crystal. The type I crystal of the free base of Compound (I) is preferably a crystal containing 90% or more of the free base type I crystal, and is more preferably a crystal containing 95% or more of the free base type I crystal.

By dissolving Compound (I) as a crude body in tetrahydrofuran and adding ethyl acetate thereto, type I crystals of the free base of Compound (I) can be obtained. However, in this case, the particle diameter obtained tends to be non-uniform. Among the obtained non-uniform crystals, for example, a flake-like portion that can be easily separated and collected with a sieve with a pore diameter of 340 µm and a powder-like portion that passes through the sieve had differences in the amounts of analogous substances and the amounts of residual solvents contained in the portions. Although both are type I crystals of the free base of Compound (I), it is more preferable to suppress the amounts of residual solvents and to produce type I crystals of the free base of Compound (I) with uniform quality and high chemical purity.

In the industrial production of pharmaceutical products, as shown in the ICH guidelines, etc., the amount of a residual solvent is required to be as small as possible. The regulatory limit for ethyl acetate is required to be 5000 ppm or less, that for tetrahydrofuran is required to be 720 ppm or less, and that for dimethyl sulfoxide is required to be 5000 ppm or less. The residual amount of ethyl acetate is preferably 5000 ppm or less, more preferably 4000 ppm or less, even more preferably 3000 ppm or less, further preferably 2000 ppm or less, and particularly preferably 1000 ppm or less. The residual amount of tetrahydrofuran is preferably 720 ppm or less, more preferably 500 ppm or less, and further preferably 300 ppm or less. The residual amount of dimethyl sulfoxide is preferably 5000 ppm or less, and more preferably 3000 ppm or less.

The type I crystal of the free base of Compound (I) according to the present disclosure can be produced, for example, by a method comprising a step (1) of dissolving Compound (I) in a solvent 1 (rich solvent), and a step (2) of adding a solvent 2 (poor solvent) to the resulting solution in the step (1) to obtain solid Compound (I).

Preferred dissolution solvents (solvent 1) used in step (1) may include dimethyl sulfoxide or tetrahydrofuran. The dissolution solvent used is more preferably dimethyl sulfoxide. Preferred solvents (solvent 2) added in the step (2) may include normal heptane, ethyl acetate or 2-propanol, when tetrahydrofuran is used in the step (1); and the preferred solvent 2 may be water, when dimethyl sulfoxide is used in the step (1). Preferably, the solvent 1 used in the step (1) is dimethyl sulfoxide, and the solvent 2 used in the step (2) is water.

In the step (2), in order to promote crystallization of crystals, an appropriate amount of the type I crystal of the free base of Compound (I) may be or may not be added as a seed crystal.

In the step (1), the temperature at which Compound (I) is dissolved can be set, as appropriate. For example, when Compound (I) is dissolved in 20 v/w dimethyl sulfoxide, the temperature is preferably room temperature to 70°C.

When dimethyl sulfoxide is used in the step (1), the type I crystals of the free base of Compound (I) can be precipitated in the step (2) by adding water while the internal temperature is set to be from room temperature to 70°C. The temperature at this time is preferably maintained at 40 C to 70 C, more preferably 60 C to 70 C.

The temperature of water added in the step (2) is more preferably 40 C to 60 C. The amount of water added at this time is preferably 3 v/w or more, more preferably 20 v/w to 25 v/w, with respect to Compound (I).

With regard to water addition time when water is used in the step (2), the water is slowly added dropwise over 0.5 to 6 hours, so that the type I crystal of the free base of Compound (I) can be obtained. As is generally known regarding crystallization, by increasing the water-adding rate, crystals with a small particle diameter can be obtained, while by decreasing the water-adding rate, crystals with a large particle diameter can be obtained.

The precipitated crystals can be isolated and purified from the suspension containing the crystals by known separation and purification means such as, for example, filtration, washing with water, drying under reduced pressure, etc.

The type II crystal of the free base of Compound (I) has, for example, the powder X-ray diffraction spectrum shown in Figure 3 and the thermogravimetry-differential thermal analysis (TG-DTA) curve shown in Figure 4.

Herein, the characteristic peaks of the type II crystal of the free base of Compound (I) in the powder X-ray diffraction spectrum (CuKα) may be, as diffraction angles (2θ±0.2°), 6.7°, 7.5°, 10.4°, 12.7°, 13.4°, 14.6°, 16.5°, 17.9°, 18.3°, 19.8°, 20.6°, 21.8°, 22.9°, 26.5° and 27.9°. More preferably, the characteristic peaks of the type II crystal of the free base of Compound (I) in the powder X-ray diffraction spectrum have diffraction angles (2θ±0.2°) of 6.7°, 7.5°, 10.4°, 12.7°, 13.4°, 14.6°, 16.5°, 17.9°, 18.3°, 19.8°, 20.6°, 21.8°, 22.9°, 26.5° and 27.9°, when measured by the reflection method.

The type II crystal of the free base of Compound (I) in one embodiment of the present disclosure has peaks at, at least, 2 diffraction angles (2θ±0.2°) selected from the following (a), and at, at least, 2 diffraction angles (2θ±0.2°) selected from the following (b), in the powder X-ray diffraction spectrum (CuKα):
(a) 10.4°, 22.9° and 27.9°; and
(b) 6.7°, 7.5°, 12.7°, 13.4°, 14.6°, 16.5°, 17.9°, 18.3°, 19.8°, 20.6°, 21.8° and 26.5°.

The type II crystal of the free base of Compound (I) in a preferred embodiment of the present disclosure has, as diffraction angles (2θ±0.2°), at least 2 peaks selected from the above-described group (a), and at least 2, preferably at least 3, more preferably at least 4, even more preferably at least 5, further preferably at least 6, still further preferably at least 7, still further preferably at least 8, still further preferably at least 9, still further preferably at least 10, still further preferably at least 11, and particularly preferably all peaks selected from the above-described group (b), in the powder X-ray diffraction spectrum. In another embodiment of the present disclosure, the type II crystal of the free base of Compound (I) may have, as diffraction angles (2θ±0.2°), all peaks selected from the above-described group (a), in the powder X-ray diffraction spectrum.

In another embodiment, the type II crystal of the free base of Compound (I) has, as diffraction angles (2θ±0.2°), all peaks selected from the above-described group (a), and at least 2, preferably at least 3, more preferably at least 4, even more preferably at least 5, further preferably at least 6, still further preferably at least 7, still further preferably at least 8, still further preferably at least 9, still further preferably at least 10, still further preferably at least 11, and particularly preferably all peaks selected from the above-described group (b), in the powder X-ray diffraction spectrum.

In addition, the endothermic peak of the type II crystal of the free base of Compound (I) in the thermogravimetry-differential thermal analysis (TG-DTA) curve may be around 232°C to 236°C, and preferably around 234°C.

In one embodiment of the present disclosure, the type II crystal of the free base of Compound (I) can be obtained, for example, by a method comprising a step (3) of adding Compound (I) to a solvent and dissolving it therein, then, a step (4) of heating and suspending the resulting solution, and then, a step (5) of cooling the solution of Compound (I) to crystallize the Compound (I). In such an embodiment, the solvent used in the step (3) may be, for example, methanol or the like. The solvent used in the step (3) is preferably methanol.

When methanol is used in the step (4), the temperature at which the heating and suspending are carried out can be 50°C to 100°C, and the reaction time can be 0.5 to 48 hours. The temperature is preferably 50°C, and the reaction time is 22 hours.

The crystallization in the step (5) may be performed under stirring. In that case, the stirring is performed using a stirrer, stirring blades, etc., as appropriate, depending on the amount of a solvent, the size of a reaction vessel, etc. In addition, the stirring operation can also be performed, for example, by shaking the reaction vessel. The stirring speed is usually 1 to 600 rpm, and preferably 10 to 100 rpm. In the step (5), the temperature for cooling may be room temperature, and the temperature is preferably room temperature.

Moreover, in the type II crystal of the free base of Compound (I) as obtained above, the chemical purity of Compound (I) is 90% or more, and it can be measured by high performance liquid chromatography (HPLC). It is preferably the type II crystal of the free base of Compound (I) in which the chemical purity of Compound (I) is 95% or more, and it is more preferably the type II crystal of the free base of Compound (I) in which the chemical purity of Compound (I) is 99% or more.

The type II crystal of the free base of Compound (I) may be any crystal, as long as it contains a free base type II crystal, and it may be either a single crystal of type II crystal of a free base, or a polymorphic mixture containing crystals other than the type II crystal. The type II crystal of the free base of Compound (I) is preferably a crystal containing 90% or more of the free base type II crystal, and is more preferably a crystal containing 95% or more of the free base type II crystal.

The type III crystal of the free base of Compound (I) has, for example, the powder X-ray diffraction spectrum shown in Figure 5 and the thermogravimetry-differential thermal analysis (TG-DTA) curve shown in Figure 6.

Herein, the characteristic peaks of the type III crystal of the free base of Compound (I) in the powder X-ray diffraction spectrum (CuKα) may be, as diffraction angles (2θ±0.2°), 6.8°, 7.5°, 8.8°, 11.7°, 12.9°, 13.9°, 14.9°, 16.7°, 17.8°, 18.8°, 19.7°, 20.7°, 22.0°, 23.4°, 24.2°, 26.2° and 26.7°. More preferably, the characteristic peaks of the type III crystal of the free base of Compound (I) in the powder X-ray diffraction spectrum have diffraction angles (2θ±0.2°) of 6.8°, 7.5°, 8.8°, 11.7°, 12.9°, 13.9°, 14.9°, 16.7°, 17.8°, 18.8°, 19.7°, 20.7°, 22.0°, 23.4°, 24.2°, 26.2° and 26.7°, when measured by the reflection method.

The type III crystal of the free base of Compound (I) in one embodiment of the present disclosure has peaks at, at least, 2 diffraction angles (2θ±0.2°) selected from the following (a), and at, at least, 2 diffraction angles (2θ±0.2°) selected from the following (b), in the powder X-ray diffraction spectrum (CuKα):
(a) 11.7°, 23.4° and 24.2°; and
(b) 6.8°, 7.5°, 8.8°, 12.9°, 13.9°, 14.9°, 16.7°, 17.8°, 18.8°, 19.7°, 20.7°, 22.0°, 26.2° and 26.7°.

The type III crystal of the free base of Compound (I) in a preferred embodiment of the present disclosure has, as diffraction angles (2θ±0.2°), at least 2 peaks selected from the above-described group (a), and at least 2, preferably at least 3, more preferably at least 4, even more preferably at least 5, further preferably at least 6, still further preferably at least 7, still further preferably at least 8, still further preferably at least 9, still further preferably at least 10, still further preferably at least 11, still further preferably at least 12, still further preferably at least 13, and particularly preferably all peaks selected from the above-described group (b), in the powder X-ray diffraction spectrum. In another embodiment of the present disclosure, the type III crystal of the free base of Compound (I) may have, as diffraction angles (2θ±0.2°), all peaks selected from the above-described group (a), in the powder X-ray diffraction spectrum.

In another embodiment, the type III crystal of the free base of Compound (I) has, as diffraction angles (2θ±0.2°), all peaks selected from the above-described group (a), and at least 2, preferably at least 3, more preferably at least 4, even more preferably at least 5, further preferably at least 6, still further preferably at least 7, still further preferably at least 8, still further preferably at least 9, still further preferably at least 10, still further preferably at least 11, still further preferably at least 12, still further preferably at least 13, and particularly preferably all peaks selected from the above-described group (b), in the powder X-ray diffraction spectrum.

In addition, the endothermic peak of the type III crystal of the free base of Compound (I) in the thermogravimetry-differential thermal analysis (TG-DTA) curve may be around 232°C to 236°C, and preferably around 234°C.

In one embodiment of the present disclosure, the type III crystal of the free base of Compound (I) can be obtained, for example, by a method comprising a step (6) of adding Compound (I) to a solvent and heating it to reflux, and then, a step (7) of quenching the reaction mixture to crystallize the Compound (I). In such an embodiment, the solvent used in the step (6) may be, for example, a single solvent selected from ethanol, 2-methyltetrahydrofuran and the like, or a mixed solvent of ethanol and 2-methyltetrahydrofuran. The solvent used in the step (6) is preferably ethanol.

The crystallization in the step (7) may be performed under stirring. In that case, the stirring is performed using a stirrer, stirring blades, etc., as appropriate, depending on the amount of a solvent, the size of a reaction vessel, etc. In addition, the stirring operation can also be performed, for example, by shaking the reaction vessel. The stirring speed is usually 1 to 600 rpm, and preferably 10 to 100 rpm.

Moreover, in the type III crystal of the free base of Compound (I) as obtained above, the chemical purity of Compound (I) is 90% or more, and it can be measured by high performance liquid chromatography (HPLC). It is preferably the type III crystal of the free base of Compound (I) in which the chemical purity of Compound (I) is 95% or more, and it is more preferably the type III crystal of the free base of Compound (I) in which the chemical purity of Compound (I) is 99% or more.

The type III crystal of the free base of Compound (I) may be any crystal, as long as it contains a free base type III crystal, and it may be either a single crystal of type III crystal of a free base, or a polymorphic mixture containing crystals other than the type III crystal. The type III crystal of the free base of Compound (I) is preferably a crystal containing 90% or more of the free base type III crystal, and is more preferably a crystal containing 95% or more of the free base type III crystal.

The crystal of the hydrochloride of Compound (I) has, for example, the powder X-ray diffraction spectrum shown in Figure 7 and the thermogravimetry-differential thermal analysis (TG-DTA) curve shown in Figure 8.

Herein, the characteristic peaks of the crystal of the hydrochloride of Compound (I) in the powder X-ray diffraction spectrum (CuKα) may be, as diffraction angles (2θ±0.2°), 6.7°, 7.4°, 11.2°, 12.6°, 14.1°, 16.6°, 20.0°, 21.1°, 22.0°, 22.6°, 23.2°, 24.5°, 26.4°, 28.0°, 30.0° and 31.6°. More preferably, the characteristic peaks of the crystal of the hydrochloride of Compound (I) in the powder X-ray diffraction spectrum have diffraction angles (2θ±0.2°) of 6.7°, 7.4°, 11.2°, 12.6°, 14.1°, 16.6°, 20.0°, 21.1°, 22.0°, 22.6°, 23.2°, 24.5°, 26.4°, 28.0°, 30.0° and 31.6°, when measured by the reflection method.

The crystal of the hydrochloride of Compound (I) according to the present disclosure is, for example, a crystal having at least 3 peaks selected from the above-described peaks, preferably a crystal having at least 4 peaks selected from the above-described peaks, more preferably a crystal having at least 5 peaks selected from the above-described peaks, even more preferably a crystal having at least 6 peaks selected from the above-described peaks, further preferably a crystal having at least 6 peaks selected from the above-described peaks, still further preferably a crystal having at least 7 peaks selected from the above-described peaks, still further preferably a crystal having at least 8 peaks selected from the above-described peaks, still further preferably a crystal having at least 9 peaks selected from the above-described peaks, still further preferably a crystal having at least 10 peaks selected from the above-described peaks, still further preferably a crystal having at least 11 peaks selected from the above-described peaks, still further preferably a crystal having at least 12 peaks selected from the above-described peaks, still further preferably a crystal having at least 13 peaks selected from the above-described peaks, still further preferably a crystal having at least 14 peaks selected from the above-described peaks, still further preferably a crystal having at least 15 peaks selected from the above-described peaks, and particularly preferably a crystal having all of the above-described peaks.

In addition, the endothermic peak of the crystal of the hydrochloride of Compound (I) in the thermogravimetry-differential thermal analysis (TG-DTA) curve may be around 239°C to 243°C, and preferably around 241°C.

In one embodiment of the present disclosure, the crystal of the hydrochloride of Compound (I) can be obtained, for example, by a method comprising a step (8) of adding Compound (I) to a solvent and then adding hydrochloric acid thereto, followed by stirring the mixture at room temperature for crystallization. In such an embodiment, the solvent that can be used in the step (8) may be, for example, ethyl acetate, methanol, ethanol, etc. or a mixed solvent thereof. The solvent used in the step (8) is preferably a mixed solvent of ethyl acetate and methanol.

The crystallization in the step (8) may be performed under stirring. In that case, the stirring is performed using a stirrer, stirring blades, etc., as appropriate, depending on the amount of a solvent, the size of a reaction vessel, etc. In addition, the stirring operation can also be performed, for example, by shaking the reaction vessel. The stirring speed is usually 1 to 600 rpm, and preferably 10 to 100 rpm.

In the step (8), the amount of hydrochloric acid added can be 0.9 to 1.1 molar equivalent, with respect to the amount of Compound (I). The amount of hydrochloric acid added is preferably 1.0 molar equivalent.

In addition, in the crystal of the hydrochloride of Compound (I) as obtained above, the chemical purity of Compound (I) is 90% or more, and it can be measured by high performance liquid chromatography (HPLC). It is preferably the crystal of the hydrochloride of Compound (I) in which the chemical purity of Compound (I) is 95% or more, and it is more preferably the crystal of the hydrochloride of Compound (I) in which the chemical purity of Compound (I) is 99% or more.

The crystal of the hydrochloride of Compound (I) may be any crystal, as long as it contains a crystal of the hydrochloride, and it may be either a single crystal of hydrochloride, or a polymorphic mixture containing crystals other than the crystal of the hydrochloride. The crystal of the hydrochloride of Compound (I) is preferably a crystal containing 90% or more of the crystal of the hydrochloride, and is more preferably a crystal containing 95% or more of the crystal of the hydrochloride.

The crystal of the maleate of Compound (I) has, for example, the powder X-ray diffraction spectrum shown in Figure 9 and the thermogravimetry-differential thermal analysis (TG-DTA) curve shown in Figure 10.

Herein, the characteristic peaks of the crystal of the maleate of Compound (I) in the powder X-ray diffraction spectrum (CuKα) may be, as diffraction angles (2θ±0.2°), 6.5°, 9.8°, 12.1°, 13.0°, 13.9°, 14.7°, 15.2°, 15.9°, 17.7°, 18.4°, 19.0°, 20.1°, 20.9°, 21.5°°, 22.9°, 23.4°, 25.3°, 28.0°, 29.7° and 30.8°. More preferably, the characteristic peaks of the crystal of the maleate of Compound (I) in the powder X-ray diffraction spectrum have diffraction angles (2θ±0.2°) of 6.5°, 9.8°, 12.1°, 13.0°, 13.9°, 14.7°, 15.2°, 15.9°, 17.7°, 18.4°, 19.0°, 20.1°, 20.9°, 21.5°, 22.9°, 23.4°, 25.3°, 28.0°, 29.7° and 30.8°, when measured by the reflection method.

The crystal of the maleate of Compound (I) according to the present disclosure is, for example, a crystal having at least 3 peaks selected from the above-described peaks, preferably a crystal having at least 4 peaks selected from the above-described peaks, more preferably a crystal having at least 5 peaks selected from the above-described peaks, even more preferably a crystal having at least 6 peaks selected from the above-described peaks, further preferably a crystal having at least 6 peaks selected from the above-described peaks, still further preferably a crystal having at least 7 peaks selected from the above-described peaks, still further preferably a crystal having at least 8 peaks selected from the above-described peaks, still further preferably a crystal having at least 9 peaks selected from the above-described peaks, still further preferably a crystal having at least 10 peaks selected from the above-described peaks, still further preferably a crystal having at least 11 peaks selected from the above-described peaks, still further preferably a crystal having at least 12 peaks selected from the above-described peaks, still further preferably a crystal having at least 13 peaks selected from the above-described peaks, still further preferably a crystal having at least 14 peaks selected from the above-described peaks, still further preferably a crystal having at least 15 peaks selected from the above-described peaks, still further preferably a crystal having at least 16 peaks selected from the above-described peaks, still further preferably a crystal having at least 17 peaks selected from the above-described peaks, still further preferably a crystal having at least 18 peaks selected from the above-described peaks, still further preferably a crystal having at least 19 peaks selected from the above-described peaks, and particularly preferably a crystal having all of the above-described peaks.

In addition, the endothermic peak of the crystal of the maleate of Compound (I) in the thermogravimetry-differential thermal analysis (TG-DTA) curve may be around 243°C to 247°C, and preferably around 245°C.

In one embodiment of the present disclosure, the crystal of the maleate of Compound (I) can be obtained, for example, by a method comprising a step (10) of adding Compound (I) and maleic acid to a solvent and then heating and stirring the resulting solution to obtain an amorphous body, then, a step (11) of heating the resulting amorphous body to reflux, and then, a step (12) of stirring the reaction mixture at room temperature and then cooling the resultant on ice for crystallization. In such an embodiment, the solvent that can be used in the step (10) may be, for example, chloroform, methanol, etc., or a mixed solvent thereof. The solvent used in the step (10) is preferably a mixed solvent of chloroform and methanol. In the step (11), as a solvent, for example, acetonitrile can be used. In the step (11), the amorphous body can be heated to reflux at a temperature of around the boiling point of the solvent, and when the solvent is acetonitrile, the applied temperature is preferably 85°C.

The maleic acid added in the step (10) must be added in the same molar equivalent as the amount of Compound (I).

The crystallization in the step (9) may be performed under stirring. In that case, the stirring is performed using a stirrer, stirring blades, etc., as appropriate, depending on the amount of a solvent, the size of a reaction vessel, etc. In addition, the stirring operation can also be performed, for example, by shaking the reaction vessel.

In addition, in the crystal of the maleate of Compound (I) as obtained above, the chemical purity of Compound (I) is 90% or more, and it can be measured by high performance liquid chromatography (HPLC). It is preferably the crystal of the maleate of Compound (I) in which the chemical purity of Compound (I) is 95% or more, and it is more preferably the crystal of the maleate of Compound (I) in which the chemical purity of Compound (I) is 99% or more.

The crystal of the maleate of Compound (I) may be any crystal, as long as it contains a crystal of the maleate, and it may be either a single crystal of maleate, or a polymorphic mixture containing crystals other than the crystal of the maleate. The crystal of the maleate of Compound (I) is preferably a crystal containing 90% or more of the crystal of the maleate, and is more preferably a crystal containing 95% or more of the crystal of the maleate.

One embodiment of obtaining the crystal forms (type I crystal, type II crystal, and type III crystal) of the free base of Compound (I), the crystal of the hydrochloride of Compound (I), and the crystal of the maleate of Compound (I) according to the present disclosure is described above, but these are merely examples. The crystal forms (type I crystal, type II crystal, and type III crystal) of the free base of Compound (I), the crystal of the hydrochloride of Compound (I), and the crystal of the maleate of Compound (I) according to the present disclosure are not limited to those obtained by the above-mentioned embodiment of obtaining them. Those skilled in the art can produce the crystal forms (type I crystal, type II crystal, and type III crystal) of the free base of Compound (I), the crystal of the hydrochloride of Compound (I), and the crystal of the maleate of Compound (I) according to the present disclosure by using known technical means.

As shown in the examples below, the type I crystal, type II crystal and type III crystal of the free base of Compound (I), the crystal of the hydrochloride of Compound (I), and the crystal of the maleate of Compound (I) according to the present disclosure have low hygroscopicity and excellent solid state stability (preservation stability, etc.). It is important for a drug substance of a pharmaceutical product and an active ingredient in a pharmaceutical product to have solid state stability in terms of both industrial operations and to maintain quality.

In addition, solvents used in the production of pharmaceutical products may have toxicity, and from the viewpoint of safety, it is desirable to have as little solvent remaining in the production process as possible. The type I crystal of the free base of Compound (I) does not contain residual solvents at levels equal to or greater than the regulation values set by the ICH (International Council for Harmonisation of Technical Requirements for Pharmaceuticals for Human Use) Guidelines.

The type I crystal, type II crystal and type III crystal of the free base of Compound (I), the crystal of the hydrochloride of Compound (I) and the crystal of the maleate of Compound (I) according to the present disclosure have excellent antitumor effects, and are useful as pharmaceutical products for treating tumors.

Upon the use of the type I crystal, type II crystal or type III crystal of the free base of Compound (I), the crystal of the hydrochloride of Compound (I), or the crystal of the maleate of Compound (I) as a medicament, various types of dosage forms can be adopted depending on the preventive or therapeutic purpose with or without crushing the crystal. Examples of the dosage form may include all of oral agents such as a tablet, a capsule, a granule, a fine granule, a powder agent, and a dry syrup agent; and parenteral agents such as a suppository, an inhalant, nasal drops, an ointment, a patch, and an injection. The type I crystal, type II crystal or type III crystal of the free base of Compound (I), the crystal of the hydrochloride of Compound (I), or the crystal of the maleate of Compound (I) is preferably utilized for oral agents. These pharmaceutical compositions can be produced by commonly used production methods that are known to skilled persons, using pharmaceutically acceptable carriers.

As pharmaceutically acceptable carriers, various types of organic or inorganic carrier substances, which are commonly used as preparation materials, are used. When the compound of the present disclosure is processed into a solid preparation, examples of the pharmaceutically acceptable carrier mixed into the compound of the present disclosure may include an excipient, a binder, a disintegrator, a lubricant, and a coating agent. When the compound of the present disclosure is processed into a liquid preparation, examples of the pharmaceutically acceptable carrier mixed into the compound of the present disclosure may include a solvent, a solubilizer, a suspending agent, a tonicity agent, a buffer, and a soothing agent. In addition, preparation additives such as an antiseptic, an antioxidant, a coloring agent, a sweetener, and a stabilizer can also be used, as necessary.

Examples of the excipient may include lactose, sucrose, D-mannitol, starch, crystalline cellulose, and calcium silicate.

Examples of the binder may include hydroxypropyl cellulose, methyl cellulose, polyvinylpyrrolidone, candy powder, and hypromellose.

Examples of the disintegrator may include sodium starch glycolate, carmellose calcium, croscarmellose sodium, crospovidone, low-substituted hydroxypropyl cellulose, and partially pregelatinized starch.

Examples of the lubricant may include talc, magnesium stearate, sucrose fatty acid ester, stearic acid, and sodium stearyl fumarate.

Examples of the coating agent may include ethyl cellulose, aminoalkyl methacrylate copolymer RS, hypromellose, and sucrose.

Examples of the solvent may include water, propylene glycol, and physiological saline.

Examples of the solubilizer may include polyethylene glycol, ethanol, α-cyclodextrin, macrogol 400, and polysorbate 80.

Examples of the suspending agent may include carrageenan, crystalline cellulose/carmellose sodium, and polyoxyethylene hydrogenated castor oil.

Examples of the tonicity agent may include sodium chloride, glycerin, and potassium chloride.

Examples of the pH adjuster/buffer may include sodium citrate, hydrochloric acid, lactic acid, phosphoric acid, and sodium dihydrogen phosphate.

Examples of the soothing agent may include procaine hydrochloride and lidocaine.

Examples of the antiseptic may include ethyl paraoxybenzoate, cresol, and benzalkonium chloride.

Examples of the antioxidant may include sodium sulfite, ascorbic acid, and natural vitamin E.

Examples of the coloring agent may include titanium oxide, iron sesquioxide, edible blue No. 1, and copper chlorophyll.

Examples of the corrigent may include aspartame, saccharin, sucralose, l-menthol, and mint flavor.

Examples of the stabilizer may include sodium pyrosulfite, sodium edetate, erythorbic acid, magnesium oxide, and dibutylhydroxytoluene.

In the case of preparing a solid preparation for oral administration, an excipient, and as necessary, a binder, a disintegrator, a lubricant, a coloring agent, a corrigent, and the like are added to the type I crystal, type II crystal or type III crystal of the free base of Compound (I), the crystal of the hydrochloride of Compound (I), or the crystal of the maleate of Compound (I), and thereafter, a tablet, a coated tablet, a granule, a powder agent, a capsule, and the like can be produced according to ordinary methods.

The amount of the type I crystal, type II crystal or type III crystal of the free base of Compound (I), the crystal of the hydrochloride of Compound (I), or the crystal of the maleate of Compound (I) to be mixed into the above-described each dosage unit form depends on the symptoms of a patient to whom the crystal should be applied, the dosage form, or the like, and thus, the amount of the compound of the present disclosure is not constant. In general, it is desirable that the applied dose is set to be about 0.05 to 1000 mg per dosage unit form in the case of an oral agent, it is set to be about 0.1 to 500 mg per dosage unit form in the case of an injection, and it is set to be about 1 to 1000 mg per dosage unit form in the case of a suppository or a topical agent.

The daily dose of the type I crystal, type II crystal or type III crystal of the free base of Compound (I), the crystal of the hydrochloride of Compound (I), or the crystal of the maleate of Compound (I) in a drug having each dosage form is different, depending on the symptoms, body weight, age, sex and the like of a patient, and thus, it cannot be generally determined. However, the crystal may be administered to an adult (body weight: 50 kg) at a daily dose of, for example, about 20 mg/day, about 40 mg/day, about 60 mg/day, about 80 mg/day, about 100 mg/day, about 200 mg/day, or about 300 mg/day, at a dose level of intermittent administration. The aforementioned crystal may also be administered at a dose level of about 400 mg/day, about 500 mg/day, about 600 mg/day, and about 3000 mg/day, about 2,500 mg/day, about 2,000 mg/day, about 1,500 mg/day, about 1,000 mg/day, about 900 mg/day, about 800 mg/day, about 700 mg/day, or any dose level in the aforementioned dose range. The crystal with the aforementioned dose is preferably administered once a day or in 2 to 3 divided doses a day.

Compound (I) or a pharmaceutically acceptable salt thereof (particularly preferably, hydrochloride or maleate) can be administered, for example, continuously (administered for 7 days per week) or intermittently, depending on pharmacokinetics and the clearance/accumulation of a drug in a specific subject. When administered intermittently, for example, a 4-day on, 3-day off (drug holiday) schedule per week may be applied, or other intermittent administration schedules that are deemed appropriate using sound medical judgment may also be applied. Continuous administration is preferable. Administration can be carried out once per day (QD) or twice or more per day (b.i.d., t.i.d., etc.), with a dose of about 20 to 960 mg/day QD being preferable. The daily dose may be administered in a single dose or in multiple individual divided doses. For example, two tablets each containing 40 mg of Compound (I) or a pharmaceutically acceptable salt thereof (particularly preferably, hydrochloride or maleate) can be administered to a subject, once per day (QD) at a total dose of 80 mg/day. In a certain embodiment, two tablets each containing 40 mg of Compound (I) or a pharmaceutically acceptable salt thereof (preferably the hydrochloride or maleate) can be administered to a subject, twice a day (b.i.d.) at a total dose of 160 mg/day. It is to be noted that the amount of Compound (I) or a pharmaceutically acceptable salt thereof administered is the amount converted to the amount of Compound (I).

Regardless of whether it is continuous administration or intermittent administration, administration continues for a specific treatment cycle, typically, at least a 28-days cycle, which can be repeated with or without drug holidays. A longer or shorter cycle can also be used, such as 14 days, 18 days, 21 days, 24 days, 35 days, 42 days, 48 days, or any range therebetween. This cycle can be repeated without or with drug holidays, depending on the subject. Other schedules can also be applied, depending on the presence or absence of adverse events, the cancer's response to treatment, patient convenience, etc. The "adverse event" refers to any undesirable or unintended illness or symptoms thereof occurring in a patient who has been administered with the drug. It does not matter whether or not there is a causal relationship with the drug. In the case of intermittent administration, administration can be performed, for example, on days 1, 3, 5, 7, 9, 11, 13, 15, 17, 19, 21, 23, 25, and 27; on days 1, 4, 8, 11, 15, 19, 23, and 27; and on days 1, 3, 5, 8, 10, 12, 15, 17, 19, 22, 24, and 26 in a 28-day cycle.

The powder X-ray diffraction measurement method performed for the crystals of Compound (I) according to the present disclosure can be measured by a reflection method.

In general, the morphology of many crystal particles tends to give selective orientation to a sample in a sample holder. The selective orientation in the sample gives influence on the intensity of various reflections, and as a result, the reflection is sometimes observed to be stronger and sometimes weaker than the reflection predicted for a completely unoriented sample.

### Examples

Hereinafter, the present disclosure will be further described specifically with reference to the Examples below. However, these Examples are not intended to limit the scope of the present disclosure. Although the present disclosure has been fully described by way of the Examples, it will be appreciated that various changes and/or modifications may be made by skilled persons. Therefore, such changes and/or modifications are included in the present disclosure unless they are deviated from the scope of the present disclosure.

### < Reagents and Measurement Methods >

Unless otherwise specified, commercially available products were used as various types of reagents in the examples. NMR spectra were measured using an AL400 (400MHz; JEOL). When tetramethylsilane was contained in a deuterated solvent, tetramethylsilane was used as an internal standard. In other cases, measurements were carried out using a non-deuterated proton peak remaining in the NMR solvent as an internal standard. All δ values were expressed in ppm.

Abbreviations have the following meanings.
s: Singlet
d: Doublet
t: Triplet
dd: Double doublet
m: Multiplet
brs: Broad singlet
DMSO-d₆: Deuterated dimethyl sulfoxide
CDCl₃: Deuterated chloroform

### Powder X-ray diffraction analysis

Powder X-ray diffraction analysis was carried out according to the following test conditions, after lightly pulverizing an appropriate amount of a test substance in an agate mortar, as needed.

Apparatus: PANalytical Empyrean
Target: Cu
X-ray output settings: 40 mA, 45 kV
Scanning range: 2.0 to 40.0°
Step size: 0.026°
Measurement method: Reflection method

Handling of the apparatuses, including data processing, was in accordance with the methods and procedures instructed for each apparatus.

It is to be noted that numerical values obtained from various spectra may vary slightly depending on the direction of crystal growth, particle size, measurement conditions, and the like. Therefore, those numerical values should not be understood strictly as they are.

### Thermogravimetry-differential thermal analysis (TG-DTA measurement)

TG-DTA measurement was carried out according to the following test conditions. Apparatus: TG/DTA7200, Hitachi High-Tech Science Corporation
Sample: Approximately 5 mg
Sample container: Made of aluminum
Temperature-increasing rate: Temperature was increased to 300°C at 10°C/minute
Atmospheric gas: Air
Nitrogen gas flow rate: 100 mL/minute

Handling of the apparatuses, including data processing, was in accordance with the methods and procedures instructed for each apparatus.

### Residual solvent measurement

Residual solvent measurement was carried out according to the following test conditions.

Measurement targets: Ethyl acetate and tetrahydrofuran

### < GC (gas chromatograph) conditions >

Apparatus: Shimadzu GC-2010 Plus
Detector: FID
Column: Inert Cap-5 Science (0.53 mm x 30 m, 5 µm)
Column temperature: 40°C for 10 minutes, increase temperature by 2°C per minute to 110°C, then increase temperature by 30°C per minute to 250°C, for 15 minutes
Injection temperature: 260°C
Detection temperature: 260°C
Atmospheric gas: He
Linear velocity: 28 cm/min
Gas flow rate: 400 mL/min
Helium flow rate: 50 mL/min
Auxiliary velocity: 50 mL/min
Auxiliary gas: He

### < HS conditions >

HS oven temperature: 80°C
HS needle temperature: 90°C
Transfer temperature: 100°C
Measurement time: 30 min
Compression time: 3/min
Pulling up time: 0.2/min
Injection time: 0.1/min
GC cycle time: 80/min
Measurement target: Dimethyl sulfoxide

### < GC (gas chromatograph) conditions >

Detector: FID
Column: Inert Cap Pure-WAX, GL Science (30 m x 0.53 mm x 5 µm)
Column temperature: 35°C for 5 minutes, increase temperature by 5°C per minute to 80°C, then increase temperature by 3°C per minute to 170°C, then increase temperature by 20°C per minute to 230°C, for 10 minutes
Injection temperature: 240°C
Detection temperature: 240°C
Atmospheric gas: He
Flow rate: 28 cm/min
Split: 10 : 1
Injection amount: 1 µL
Detection gas flow rate: H2: 50 ml/min, Air: 400 mL/min, N2: 45 mL/min

Handling of the apparatuses, including data processing, was in accordance with the methods and procedures instructed for each apparatus.

### Example 1 Production of type I crystal of free base of Compound (I)

Compound (I) (11.7 g) that had been synthesized according to the method described in International Publication WO2017/200087 (Example 32) was suspended in ethyl acetate (110 mL), and the obtained mixture was stirred at 100°C for 4 hours and was then stirred at room temperature for 18 hours. The suspension was filtrated, and the resulting powders (10.4 g) were re-suspended in ethyl acetate (220 mL). The suspension was stirred at 100°C for 5 hours, and was then stirred at room temperature for 9 hours. The suspension was filtrated to obtain a type I crystal of the free base of Compound (I) (9.37 g) at a yield of 80.1% and at a chemical purity of 97.31%.

The ¹H-NMR spectrum of the obtained type I crystal of Compound (I) was as follows.
¹H-NMR (DMSO-D6) δ: 8.23 (1H, s), 7.92 (1H, d, J = 8.8 Hz), 7.09 (1H, d, J = 8.8 Hz), 6.45 (1H, t, J = 5.2 Hz), 4.49 (2H, d, J = 13.2 Hz), 3.22 (5H, m, 3.29-3.13), 2.71 (2H, t, J = 6.7 Hz), 1.93 (2H, d, J = 12.0 Hz), 1.76 (2H, m, 1.82-1.70), 1.39 (6H, s), 1.03 (9H, s)

Moreover, from the ¹H-NMR spectrum, it was calculated that about 5700 ppm ethyl acetate was contained as a residual solvent. The amount of the residual solvent from the ¹H-NMR spectrum was calculated by converting the integral value ratio between the peak derived from Compound (I) and the solvent peak into a weight ratio using the molecular weight, and then calculating it as weight of the solvent ÷ (weight of Compound (I) + weight of the solvent).

The powder X-ray diffraction spectrum and thermogravimetry-differential thermal analysis (TG-DTA) curve of the obtained type I crystal of the free base are as follows.

The powder X-ray diffraction spectrum is shown in Figure 1.

Characteristic diffraction angles are as follows.

Characteristic diffraction angles (2θ±0.2°):
6.2°, 6.7°, 8.3°, 8.7°, 9.3°, 9.7°, 11.1°, 13.0°, 13.7°, 15.4°, 16.3°, 16.9°, 17.7°, 18.7°, 19.4°, 20.3°, 25.1°, 25.9°, and 26.5°.

Individual peaks are as shown in the following table (Table 1).

### [Table 1]

**Table 1**

| Peak position (°2θ) | Intensity (cts) | Peak position (°2θ) | Intensity (cts) |
|---|---|---|---|
| 6.2 | 554.1 | 25.1 | 947.3 |
| 6.7 | 413.2 | 25.4 | 578.1 |
| 8.3 | 538.2 | 25.9 | 868.6 |
| 8.7 | 383.4 | 26.5 | 746.0 |
| 9.3 | 1789.2 | 27.1 | 198.9 |
| 9.7 | 586.9 | 27.6 | 197.2 |
| 10.3 | 139.5 | 27.9 | 54.8 |
| 11.1 | 1477.5 | 28.3 | 208.7 |
| 12.5 | 421.8 | 29.0 | 91.2 |
| 13.0 | 973.0 | 29.2 | 65.8 |
| 13.7 | 675.3 | 29.9 | 180.8 |
| 14.0 | 381.0 | 30.9 | 113.0 |
| 15.4 | 1308.0 | 31.4 | 25.9 |
| 16.3 | 7208.6 | 32.9 | 88.7 |
| 16.9 | 599.8 | 34.5 | 59.5 |
| 17.7 | 811.9 | 35.3 | 22.9 |
| 18.3 | 194.0 | 36.0 | 29.7 |
| 18.7 | 872.3 | 36.5 | 100.7 |
| 19.4 | 1669.6 | 36.9 | 39.7 |
| 20.3 | 2021.3 | 37.7 | 14.9 |
| 20.8 | 563.4 | 38.0 | 18.5 |
| 21.3 | 443.1 | 38.5 | 13.9 |
| 21.9 | 591.3 | 38.9 | 17.8 |
| 23.0 | 464.8 | 39.4 | 53.6 |
| 23.6 | 191.6 | | |
| 24.0 | 151.5 | | |
| 24.7 | 115.3 | | |

The thermogravimetry-differential thermal analysis (TG-DTA) curve is shown in Figure 2.

### Production Example 1 Production of type I crystal of free base of Compound (I)

The type I crystal of the free base of Compound (I) can also be produced by the following method.

Compound (I) (6.0 g) that had been synthesized according to the method described in International Publication WO2017/200087 (Example 32) was dissolved in a mixed solvent of tetrahydrofuran (42 mL) and ethyl acetate (210 mL) by stirring for 1 hour under heat reflux in an oil bath at 110°C. At this time, the internal temperature was 75°C. The oil bath was removed, and the suspension was stirred at room temperature for 3 hours to crystallize Compound (I). At this time, the internal temperature of the suspension was 30°C. The suspension was filtrated, and was dried under reduced pressure at 65°C for 10 hours to obtain the type I crystal of the free base of Compound (I) (3.83 g; yield: 64%).

The obtained Compound (I) (20.00 g) was suspended in dimethyl sulfoxide (400 mL, 20 v/w) under stirring, followed by deaeration at room temperature for 5 minutes, and nitrogen gas was sealed therein. The obtained suspension was heated and stirred in an oil bath at 65°C for 25 minutes to dissolve the crude Compound (I) to obtain a light yellow solution. At this time, the internal temperature at this time was 59°C.

The present solution was subjected to clear filtration through a Glass-Fiber Filter (Whatman, GF/B, Φ60mm), and the container was fully washed with 20 mL (1 v/w) of dimethyl sulfoxide. At this time, the temperature of the filtrate at this time was 40°C.

The obtained filtrate was heated and stirred again in an oil bath at 65°C until the internal temperature reached 58°C, and water (420 mL, 21 v/w) was then added to the reaction mixture for crystallization. Crystallization from Compound (I) was initiated around when water (70 mL, 3.5 v/w) was added dropwise over 4 hours, and the internal temperature at that time was around 63°C.

Thereafter, water (350 mL, 17.5 v/w) was added dropwise thereto over 70 minutes to further promote crystallization. After addition of the water had been completed, the suspension was stirred for further 1 hour at an internal temperature of 67°C, and thereafter, the suspension was allowed to cool to room temperature over 3 hours under stirring.

The resulting suspension was filtrated through Kiriyama filter papers (2 sheets, No. 3, Φ95 mm (top) and No. 4, Φ95 mm (bottom)), and the filtrated wet crystals of Compound (I) were washed with water (300 mL, 15 v/w).

The resulting wet crystals of the type I crystal of Compound (I) were dried under reduced pressure at 70°C for 14 hours to obtain a type I crystal of the free base of Compound (I) (19.13 g; yield: 96%; chemical purity 99.12%), which was light yellow in color. From the 1H-NMR spectrum, the residual solvent was calculated to be about 2400 ppm of dimethyl sulfoxide, but ethyl acetate and tetrahydrofuran were not detected. The amount of the residual solvent was calculated in the same manner as that in Example 1. Furthermore, when the residual solvent was subjected to GC analysis, the results were dimethyl sulfoxide (2629 ppm), tetrahydrofuran (ND), and ethyl acetate (ND). It is to be noted that ND means below the limit of quantification, and the values below the limit of quantification for each solvent in the GC analysis were set to be 235 ppm dimethyl sulfoxide, 900 ppm ethyl acetate, and 145 ppm tetrahydrofuran.

### Example 2 Production of type II crystal of free base of Compound (I)

40 mL of Methanol was added to 2.00 g of Compound (I) that had been synthesized according to the method described in International Publication WO2017/200087, and Compound (I) was suspended in the methanol by heating and stirring at 50°C for 22 hours. After that, the suspension was cooled and stirred at room temperature for 2 hours, and a solid was then collected by filtration. The solid was dried under reduced pressure at 40°C for 20 hours to obtain 1.883 g of type II crystals of the free base of Compound (I) (yield: 94%).

The ¹H-NMR spectrum of the obtained type II crystal of the free base of Compound (I) was as follows.

¹H-NMR (DMSO-d6) δ: 8.22 (1H, s), 7.91 (1H, d, J = 8.8 Hz), 7.09 (1H, d, J = 8.8 Hz), 6.41 (1H, t, J = 5.2 Hz), 4.49 (2H, d, J = 13.6 Hz), 3.30-3.14 (5H, m),2.71 (2H, t, J = 6.7 Hz), 1.92 (2H, d, J = 12.0 Hz), 1.80-1.70 (2H, m), 1.39 (6H, s), 1.03 (9H, s).

The powder X-ray diffraction spectrum and thermogravimetry-differential thermal analysis (TG-DTA) curve of the obtained type II crystal of the free base are as follows.

The powder X-ray diffraction spectrum is shown in Figure 3.

Characteristic diffraction angles are as follows.

Characteristic diffraction angles (2θ±0.2°): The diffraction angles (2θ±0.2°) are 6.7°, 7.5°, 10.4°, 12.7°, 13.4°, 14.6°, 16.5°, 17.9°, 18.3°, 19.8°, 20.6°, 21.8°, 22.9°, 26.5°, and 27.9°.

Individual peaks are as shown in the following table (Table 2).

### [Table 2]

**Table 2**

| Peak position (°2θ) | Intensity (cts) |
|---|---|
| 3.6 | 87.6 |
| 6.7 | 1672.4 |
| 7.5 | 1513.6 |
| 8.5 | 135.2 |
| 10.4 | 634.7 |
| 12.7 | 297.3 |
| 13.4 | 389.4 |
| 14.6 | 916.0 |
| 15.9 | 99.4 |
| 16.5 | 857.4 |
| 17.1 | 94.1 |
| 17.9 | 349.1 |
| 18.3 | 256.7 |
| 19.0 | 65.7 |
| 19.8 | 605.3 |
| 20.6 | 215.8 |
| 21.8 | 301.1 |
| 22.4 | 108.1 |
| 22.9 | 418.5 |
| 24.0 | 123.4 |
| 24.2 | 76.3 |
| 24.7 | 95.2 |
| 25.9 | 61.1 |
| 26.5 | 409.5 |
| 27.3 | 62.1 |
| 27.9 | 260.1 |
| 29.8 | 109.0 |
| 31.0 | 89.3 |
| 32.2 | 28.6 |
| 34.2 | 21.6 |
| 35.4 | 40.3 |
| 38.9 | 24.3 |

The thermogravimetry-differential thermal analysis (TG-DTA) curve is shown in Figure 4.

### Example 3 Production of type III crystal of free base of Compound (I)

28 mL of Ethanol was added to 500 mg of Compound (I) that had been synthesized according to the method described in International Publication WO2017/200087, and the obtained mixture was heated to reflux around an internal temperature of 78°C. After confirming dissolution of the compound, the reaction solution was quenched in a dry icemethanol bath (the internal temperature became -2°C about 1 minute after initiation of the cooling). After confirming precipitation of crystals, the bath was changed to an ice bath, the solution was then stirred around an internal temperature of 0°C for 30 minutes, and a solid was then collected by filtration. The solid was dried under reduced pressure at 40°C for 20 hours to obtain 347 mg of type III crystal of the free base of Compound (I) (yield: 69%).

The ¹H-NMR spectrum of the obtained type III crystal of the free base of Compound (I) was as follows.

¹H-NMR (DMSO-d6) δ: 8.23 (1H, s), 7.92 (1H, d, J = 8.8 Hz), 7.10 (1H, d, J = 8.8 Hz), 6.44 (1H, t, J = 5.2 Hz), 4.50 (2H, d, J = 13.6 Hz), 3.41-3.14 (5H, m), 2.73 (2H, t, J = 6.7 Hz), 1.94 (2H, d, J = 12.0 Hz), 1.85-1.72 (2H, m), 1.40 (6H, s), 1.05 (9H, s).

The powder X-ray diffraction spectrum and thermogravimetry-differential thermal analysis (TG-DTA) curve of the obtained type III crystal of the free base are as follows.

The powder X-ray diffraction spectrum is shown in Figure 5.

Characteristic diffraction angles are as follows.

Characteristic diffraction angles (2θ±0.2°): The diffraction angles (2θ±0.2°) are 6.8°, 7.5°, 8.8°, 11.7°, 12.9°, 13.9°, 14.9°, 16.7°, 17.8°, 18.8°, 19.7°, 20.7°, 22.0°, 23.4°, 24.2°, 26.2°, and 26.7°.

Individual peaks are as shown in the following table (Table 3).

### [Table 3]

**Table 3**

| Peak position (°2θ) | Intensity (cts) |
|---|---|
| 2.9 | 107.7 |
| 6.8 | 834.4 |
| 7.5 | 1302.6 |
| 8.8 | 184.9 |
| 10.4 | 112.6 |
| 11.7 | 408.5 |
| 12.9 | 227.1 |
| 13.4 | 78.6 |
| 13.9 | 153.7 |
| 14.9 | 681.0 |
| 16.7 | 457.0 |
| 17.8 | 391.2 |
| 18.8 | 118.1 |
| 19.7 | 147.3 |
| 20.7 | 370.2 |
| 22.0 | 710.6 |
| 22.9 | 104.5 |
| 23.4 | 151.4 |
| 24.2 | 228.2 |
| 26.2 | 157.7 |
| 26.7 | 196.5 |
| 27.8 | 47.2 |
| 28.8 | 73.1 |
| 29.8 | 80.2 |
| 30.6 | 67.0 |
| 32.1 | 26.9 |
| 33.8 | 21.6 |
| 35.1 | 31.1 |
| 38.0 | 15.9 |

The thermogravimetry-differential thermal analysis (TG-DTA) curve is shown in Figure 6.

### Example 4 Production of crystal of hydrochloride of Compound (I)

3 ml of Ethyl acetate and 3 ml of methanol were added to Compound (I) (202 mg) that had been synthesized according to the method described in International Publication WO2017/200087, and the obtained mixture was then stirred. Thereafter, 71 µl of 5 M hydrochloric acid aqueous solution (1 molar equivalent with respect to Compound (I)) was added, and the obtained solution was then stirred at room temperature for 2 hours. Thereafter, a solid was collected by filtration and was then dried under reduced pressure to obtain a crystal of the hydrochloride of Compound (I) (161 mg) (yield: 75%).

The ¹H-NMR spectrum of the obtained crystal of the hydrochloride of Compound (I) was as follows.
¹H-NMR (DMSO-d6) δ: 11.10 (1H, s), 8.96 (1H, br s), 8.22 (1H, s), 7.96 (1H, d, J = 8.8 Hz), 7.18 (1H, d, J = 8.8 Hz), 6.79 (1H, br s), 4.48 (2H, d, J = 13.6 Hz), 3.35-3.19 (5H, m), 2.71 (2H, t, J = 6.7 Hz), 1.95-1.75 (4H, m), 1.39 (6H, s), 1.31 (9H, s).

The powder X-ray diffraction spectrum and thermogravimetry-differential thermal analysis (TG-DTA) curve of the obtained crystal of the hydrochloride are as follows.

The powder X-ray diffraction spectrum is shown in Figure 7.

Characteristic diffraction angles are as follows.

Characteristic diffraction angles (2θ±0.2°): 6.7°, 7.4°, 11.2°, 12.6°, 14.1°, 16.6°, 20.0°, 21.1°, 22.0°, 22.6°, 23.2°, 24.5°, 26.4°, 28.0°, 30.0°, and 31.6°.

The thermogravimetry-differential thermal analysis was measured according to the test conditions for TG-DTA measurement described in the above section "< Reagents and Measurement Methods >. However, regarding the temperature-increasing rate, the temperature was increased at 10°C/min to 400°C.

Individual peaks are as shown in the following table (Table 4).

### [Table 4]

**Table 4**

| Peak position (°2θ) | Intensity (cts) |
|---|---|
| 6.7 | 62.6 |
| 7.4 | 75.8 |
| 8.1 | 4.8 |
| 10.4 | 192.9 |
| 11.2 | 70.3 |
| 12.6 | 113.7 |
| 13.5 | 12.6 |
| 14.1 | 153.1 |
| 15.1 | 15.9 |
| 16.6 | 84.6 |
| 17.8 | 18.0 |
| 18.3 | 18.5 |
| 20.0 | 58.9 |
| 21.1 | 53.9 |
| 22.0 | 54.2 |
| 22.6 | 26.3 |
| 23.2 | 57.7 |
| 24.5 | 56.9 |
| 26.4 | 40.4 |
| 26.8 | 16.0 |
| 27.2 | 16.6 |
| 28.0 | 19.9 |
| 28.4 | 11.9 |
| 30.0 | 20.4 |
| 30.4 | 15.5 |
| 31.6 | 22.4 |
| 32.0 | 6.3 |
| 33.7 | 5.6 |
| 34.6 | 9.5 |
| 35.5 | 3.4 |

The thermogravimetry-differential thermal analysis (TG-DTA) curve is shown in Figure 8.

### Example 5 Production of crystal of maleate of Compound (I)

20 mL of Chloroform/methanol (9/1) was added to Compound (I) (2.0 g) that had been synthesized according to the method described in International Publication WO2017/200087, and the compound was dissolved in the solvent. To the obtained solution, 405 mg of maleic acid (1 molar equivalent with respect to Compound (I)) was added, and the obtained mixture was then heated and stirred at to 45°C. After confirming dissolution of the compound, the solvent was distilled away under reduced pressure, and the residue was dried under reduced pressure at 40°C to obtain 2.43 g of an amorphous body of maleate (yield: 101%, CHCl3 remained). To 500 mg of the amorphous body of maleate, 200 mL of acetonitrile was added, and the obtained mixture was then heated to reflux for dissolution. The reaction solution was allowed to cool and was stirred at room temperature, and after the internal temperature had returned to around room temperature, the reaction solution was cooled on ice and was stirred for 1 hour. Thereafter, a solid was collected by filtration. The solid was dried under reduced pressure at 40°C to obtain 332 mg of a crystal of the maleate (yield: 66%).

The ¹H-NMR spectrum of the obtained crystal of the maleate of Compound (I) was as follows.

1H-NMR (DMSO-d6) δ: 11.12 (1H, s), 8.39(1H, s), 7.99 (1H, d, J = 8.8 Hz), 7.14 (1H, d, J = 8.8 Hz), 6.55 (1H, t, J = 5.2 Hz), 6.00 (2H, s), 4.50 (2H, d, J = 13.6 Hz), 3.60-3.07 (7H, m), 1.91 (2H, d, J = 12.0 Hz), 1.80-1.70 (2H, m), 1.39 (6H, s), 1.03 (9H, s).

The powder X-ray diffraction spectrum and thermogravimetry-differential thermal analysis (TG-DTA) curve of the obtained crystal of the maleate are as follows.

The powder X-ray diffraction spectrum is shown in Figure 9.

Characteristic diffraction angles are as follows.

Characteristic diffraction angles (2θ±0.2°): 6.5°, 9.8°, 12.1°, 13.0°, 13.9°, 14.7°, 15.2°, 15.9°, 17.7°, 18.4°, 19.0°, 20.1°, 20.9°, 21.5°, 22.9°, 23.4°, 25.3°, 28.0°, 29.7°, and 30.8°.

Individual peaks are as shown in the following table (Table 5).

### [Table 5]

**Table 5**

| Peak position (°2θ) | Intensity (cts) |
|---|---|
| 6.5 | 269.6 |
| 7.7 | 15.9 |
| 9.8 | 138.4 |
| 10.9 | 19.7 |
| 12.1 | 52.4 |
| 13.0 | 53.5 |
| 13.9 | 60.6 |
| 14.7 | 93.7 |
| 15.2 | 245.2 |
| 15.9 | 27.6 |
| 17.7 | 91.3 |
| 18.4 | 136.8 |
| 19.0 | 51.5 |
| 20.1 | 112.1 |
| 20.9 | 98.0 |
| 21.5 | 178.3 |
| 22.9 | 120.8 |
| 23.4 | 63.6 |
| 24.4 | 17.7 |
| 25.3 | 111.9 |
| 26.2 | 25.6 |
| 28.0 | 74.5 |
| 28.6 | 19.2 |
| 29.7 | 35.0 |
| 30.8 | 34.4 |
| 31.2 | 16.4 |
| 32.6 | 16.8 |
| 33.6 | 14.6 |
| 36.9 | 8.8 |

The thermogravimetry-differential thermal analysis was measured according to the test conditions for TG-DTA measurement described in the above section < Reagents and Measurement Methods >. However, regarding the temperature-increasing rate, the temperature was increased at 10°C/min to 400°C.

The thermogravimetry-differential thermal analysis (TG-DTA) curve is shown in Figure 10.

### Comparative Example 1 Production of crystal of 1/2 malate of Compound (I)

75 mL of 1-Propanol was added to 1.00 g of Compound (I) that had been synthesized according to the method described in International Publication WO2017/200087, and the obtained mixture was heated and stirred, so that the compound was dissolved therein around an internal temperature of 75°C. 129 mg (0.55 eq.) of L-malic acid was added to the obtained solution, and the mixed solution was then fully washed with 5 mL of 1-propanol. The internal temperature was increased up to 90°C, and the reaction mixture was the stirred at the same temperature as described above for 30 minutes. Thereafter, the reaction mixture was allowed to gradually cool at room temperature and was stirred. Crystals were precipitated around 70°C. After stirring for 30 minutes while maintaining the same temperature as described above, the reaction mixture was cooled and was stirred overnight at 10°C or lower. Thereafter, a solid was collected by filtration. The solid was dried under reduced pressure at 40°C for 2 days to obtain 1.00 g of 1/2 malate (yield: 90%).

The ¹H-NMR spectrum of the obtained 1/2 malate of Compound (I) was as follows.

¹H-NMR (DMSO-d6) δ: 8.22 (1H, s), 7.94 (1H, d, J = 8.8 Hz), 7.11 (1H, d, J = 8.8 Hz), 6.54 (1H, t, J = 5.2 Hz), 4.49 (2H, d, J = 12.8 Hz), 3.85-3.80 (0.5H, m), 3.37-3.16 (5H, m), 2.88 (2H, br s), 2.51-2.26 (1H, m ), 1.93-1.72 (4H, m ), 1.41 (6H, s), 1.15 (9H, s).

The powder X-ray diffraction spectrum and thermogravimetry-differential thermal analysis (TG-DTA) curve of the obtained crystal of the 1/2 malate of Compound (I) are as follows.

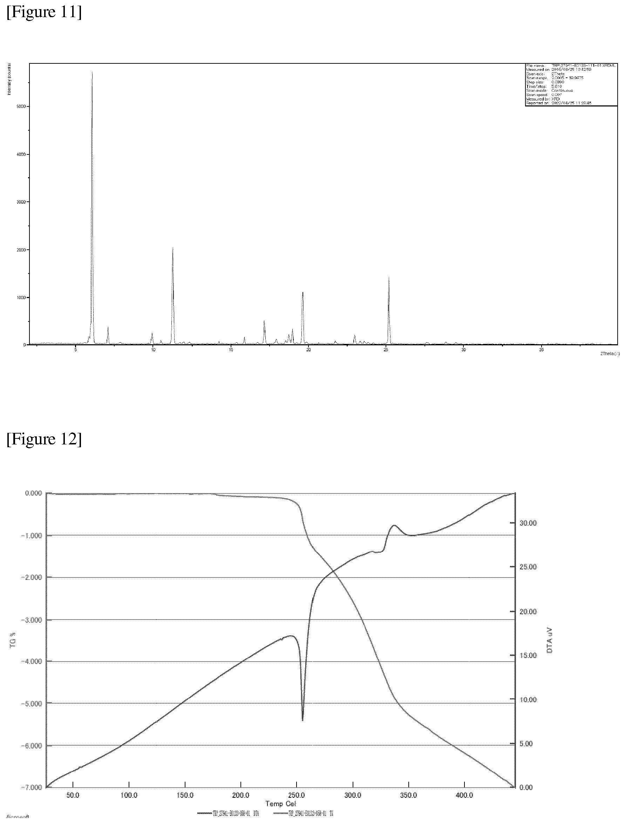

The powder X-ray diffraction spectrum is shown in Figure 11.

Characteristic diffraction angles are as follows.

Characteristic diffraction angles (2θ±0.2°): 6.1°, 7.1°, 10.0°, 11.2°, 17.2°, 19.6°, 16.6°, and 25.2°.

The thermogravimetry-differential thermal analysis (TG-DTA) curve is shown in Figure 12.

### Comparative Example 2 Production of crystal of acetate of Compound (I)

1 mL of Butyl acetate and 998 µL of acetic acid (1 molar equivalent with respect to Compound (I)) were added to 1.00 g of Compound (I) that had been synthesized according to the method described in International Publication WO2017/200087, and the obtained mixture was then stirred at room temperature for dissolution.

The temperature of the obtained solution was increased to 50°C, and 9 mL of butyl acetate was added thereto. The obtained mixture was stirred for 1 hour, and a solid was then precipitated. The reaction mixture was allowed to cool, as was, to room temperature, and thereafter, it was stirred under cooling on ice for 1 hour, and a solid was collected by filtration. The solid was dried under reduced pressure at 40°C for 20 hours to obtain 693 mg of acetate (yield: 63%).

The ¹H-NMR spectrum of the obtained acetate of Compound (I) was as follows. ¹H-NMR (DMSO-d6) δ: 8.22 (1H, s), 7.91 (1H, d, J = 8.8 Hz), 7.09 (1H, d, J = 8.8 Hz), 6.53 (1H, t, J = 5.2 Hz), 4.48 (2H, d, J = 12.8 Hz), 3.30-3.13 (5H, m), 2.74 (2H, t, J = 6.7 Hz), 1.88-1.7 (7H, m), 1.38 (6H, s), 1.05 (9H, s).

The powder X-ray diffraction spectrum and thermogravimetry-differential thermal analysis (TG-DTA) curve of the obtained acetate of Compound (I) are as follows.

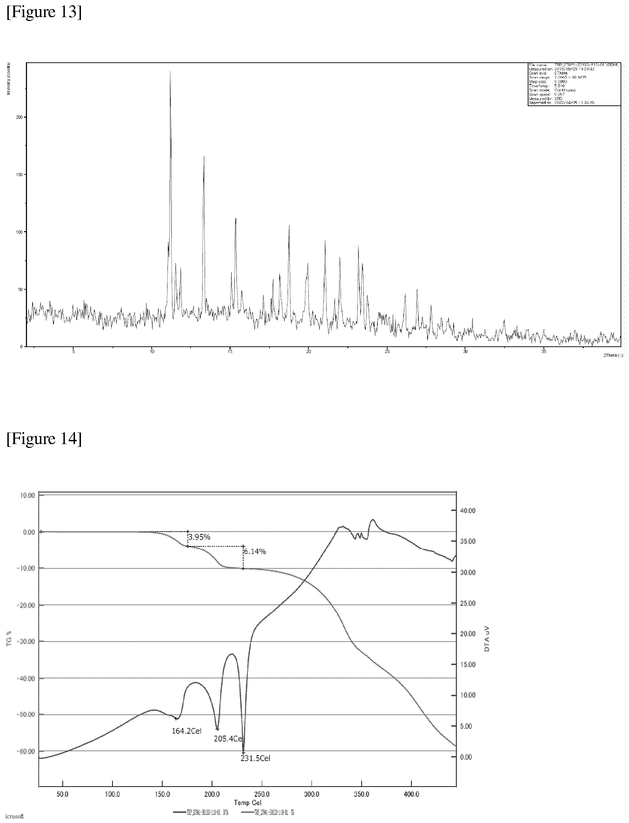

The powder X-ray diffraction spectrum is shown in Figure 13.

Characteristic diffraction angles are as follows.

Characteristic diffraction angles (2θ±0.2°): 11.2°, 11.5°, 12.6°, 13.3°, 15.4°, 15.8°, 17.1°, 17.7°, 18.2°, 18.8°, 19.9°, 21.1°, 21.7°, 22.0°, 23.2°, 23.4°, 23.8°, 26.2°, 26.9°, and 27.8°.

The thermogravimetry-differential thermal analysis (TG-DTA) curve is shown in Figure 14.

### Test Example 1 Solid state stability test

The preservation stability of each of the type I crystal, type II crystal, and type III crystal of the free base of Compound (I), which were produced according to the methods described in Examples 1, 2 and 3 above, respectively, was tested under the following conditions.
Preservation conditions: 70°C/75% RH (open system and closed system)
Measuring point: 2 weeks
Preserved amount: about 50 mg
Preservation container: brown glass bottle

The powder X-ray diffraction of the sample after preservation was measured according to the aforementioned method. A change in the amount of analogous substances (the amount of substances detected other than Compound (I)) was measured by weighing out about 1 mg of the sample, dissolving it in about 5 mL of a water/acetonitrile mixed solution (1 : 1), accurately measuring 5 µL of this solution, and analyzing it by HPLC using the following method.

Column: InertSustain C18, 4.6 x 150 mm, 3 µm
Column temperature: 40°C
Column flow rate: 1.0 mL/min
Mobile phase: A - 10 mM phosphate buffer (pH 5.5)/acetonitrile (9 : 1); B - acetonitrile
Detection UV: 220 nm
Gradient:

| Time (min) | A | B |
|---|---|---|
| 0 to 20 | 100%→40% | 0%→60% |
| 20 to 27 | 40% | 60% |
| 27 to 28 | 40%→100% | 60%→0% |
| 28 to 35 | 95% | 5% |

As a result, no change was observed in the powder X-ray diffraction patterns of the type I crystal, type II crystal and type III crystal of the free base, and they were found to be extremely stable crystals. In addition, as shown in Table 6 below, the amount of analogous substances in each crystal after preservation was small, and no change was observed in appearance.

### [Table 6]

**Table 6**

| | Total amount of analogous substances (%) | | | Powder X-ray diffraction pattern | Appearance change |
|---|---|---|---|---|---|
| | Initial value | 70°C/75% RH (open system) | 70°C/75% RH (closed system) | | |
| Free body type I crystal | 0.6% | 0.5% | 0.5% | No significant change | No significant change |
| Free body type II crystal | 0.5% | 0.6% | 0.5% | No significant change | No significant change |
| Free body type III crystal | 0.5% | 0.8% | 0.7% | No significant change | No significant change |

### Test Example 2 Dynamic moisture adsorption/desorption test

For the test, a VTI-SA+ (TA Instruments) was used. About 10 mg of each of a free base type I crystal, a free base type II crystal, and a free base type III crystal was weighed, and was placed in a pan. While confirming that the weight fluctuation was within 0.0100% in 5 minutes, the temperature was raised up to 60°C at 1 degree per minute. If the weight fluctuated, the temperature was maintained for a maximum of 5 hours before proceeding to the next step. Thereafter, the temperature was lowered to 25°C, and the humidity was raised from 5% RH to 95% RH, and was then lowered to 5% RH. At that time, the humidity was changed by 5% RH while confirming that the weight fluctuation was within 0.00100% in 5 minutes. If the weight fluctuated, the humidity was maintained for a maximum of 2 hours before proceeding to the next step.

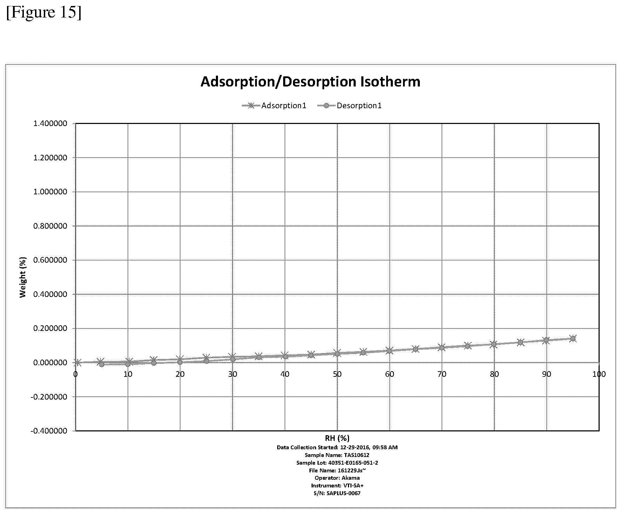

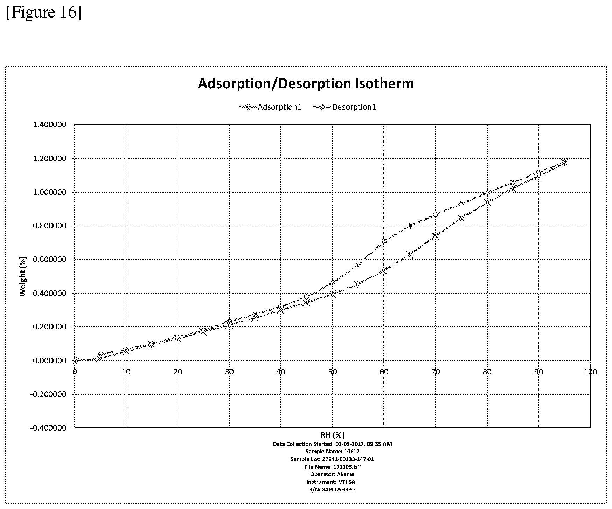

As shown in Figure 15, Figure 16, and Figure 17, the free base type I crystal, the free base type II crystal, and the free base type III crystal could be confirmed to have low hygroscopicity. It became clear that, in particular, the type I crystal and type III crystal of the free base exhibit excellent low hygroscopicity.

### Text Example 3 Heat stability test

Using the free base type I crystal, the free base type II crystal, and the free base type III crystal, which were produced according to the methods described in Examples 1, 2 and 3 above, a heat stability test was carried out.

Powder X-ray diffraction measurement was carried out at 25°C, and the temperature was raised to 50°C over 30 minutes, after which powder X-ray diffraction measurement was carried out. Thereafter, the temperature was further raised to 210°C at 10°C/10 minutes, and powder X-ray diffraction measurement was then carried out. After that, the temperature was lowered to 25°C over 30 minutes, and powder X-ray diffraction measurement was then carried out. In the obtained spectra, whether new peaks appeared in the target spectrum before and after initiation of the test, or the presence or absence of decrease in the peaks was confirmed.

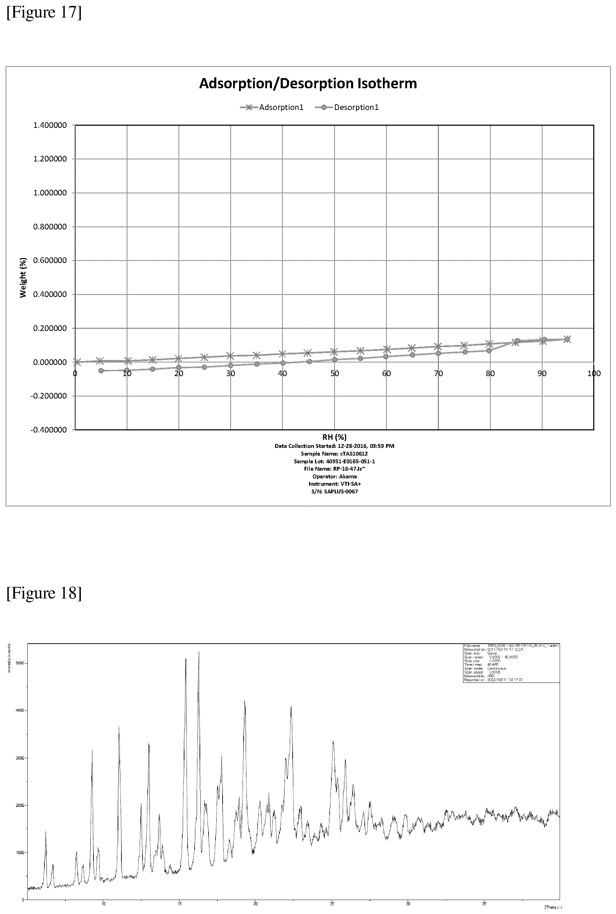

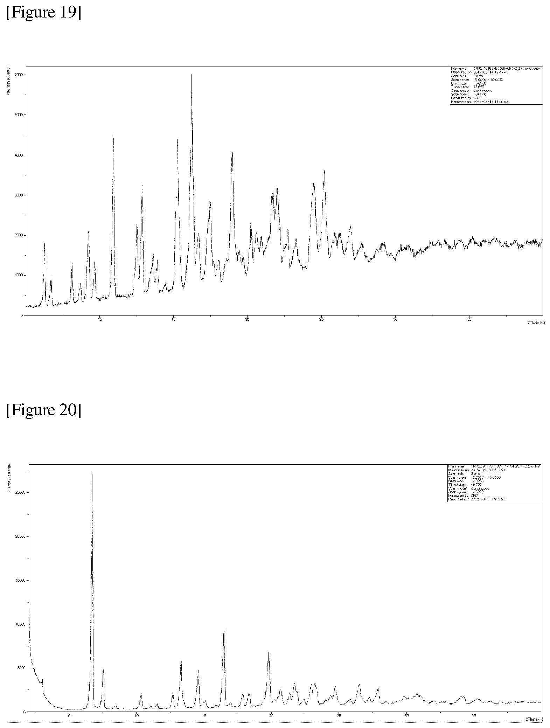

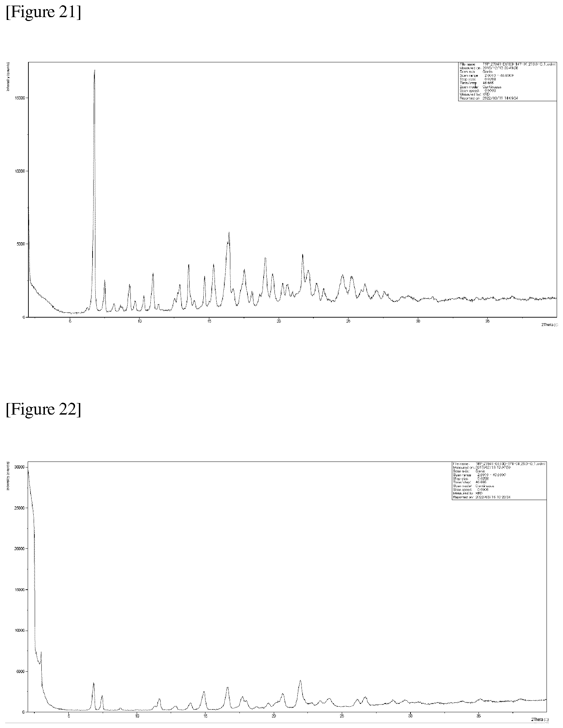

The results of the heat stability test performed at 25°C and 210°C on the free base type I crystal, the free base type II crystal, and the free base type III crystal are shown in Figures 18, 19, 20, 21, 22, and 23, respectively.

As a result, no changes were observed in the crystal form of the free base type I crystal under heating to 210°C, and it was confirmed that the type I crystal was stable. On the other hand, since new peaks appeared in the free base type II crystal and the free base type III crystal by heating, it was found that the crystal forms are not retained.

### Test Example 4 Solid state stability test

The crystal of the hydrochloride of Compound (I), the crystal of the maleate of Compound (I), the crystal of 1/2 malate of Compound (I), and the crystal of the acetate of Compound (I),which were produced according to the methods described in Example 4, Example 5, Comparative Example 1, and Comparative Example 2, were tested in terms of preservation stability under the conditions of Test Example 1.

As a result, both the crystal of the hydrochloride of Compound (I) and the crystal of the maleate of Compound (I) showed no changes in the powder X-ray diffraction patterns, and it was found that these crystals are extremely stable crystals. In addition, as shown in Table 7 and Table 8 below, both the crystal of the hydrochloride of Compound (I) and the crystal of the maleate of Compound (I) had only small amounts of analogous substances after preservation, and no change was observed in appearance.

As shown in Table 9 and Table 10 below, the crystal of the 1/2 malate of Compound (I) and the crystal of the acetate of Compound (I) showed no change in appearance after preservation, but had large amounts of analogous substances.

**[Table 7]**

| Table 7 Crystal of hydrochloride | | | | |
|---|---|---|---|---|
| Total amount of analogous substances (%) | | | Powder X-ray diffraction pattern | Appearance change |
| Initial value | 70°C/75% RH (open system) | 70°C/75% RH (closed system) | | |
| 0.2% | 0.2% | 0.2% | No significant change | No significant change |

**[Table 8]**

| Table 8 Crystal of maleate | | | | |
|---|---|---|---|---|
| Total amount of analogous substances (%) | | | Powder X-ray diffraction pattern | Appearance cha |
| Initial value | 70°C/75% RH (open system) | 70°C/75% RH (closed system) | | |
| 0.1% | 0.1% | 0.1% | No significant change | No signifi change |

**[Table 9]**

| Table 9 Crystal of 1/2 malate | | | |
|---|---|---|---|
| Total amount of analogous substances (%) | | | Appearance cha |
| Initial value | 70°C/75% RH (open system) | 70°C/75% RH (closed system) | |
| 0.4% | 64.0% | 5.2% | No signifi change |

**[Table 10]**

| Table 10 Crystal of acetate | | | |
|---|---|---|---|
| Total amount of analogous substances (%) | | | Appearance change |
| Initial value | 70°C/75% RH (open system) | 70°C/75% RH (closed system) | |
| 0. 3% | 1. 1% | 0. 3% | No significant change |

### Test Example 5 Dynamic moisture adsorption/desorption test

The crystal of the hydrochloride of Compound (I), the crystal of the maleate of Compound (I), the crystal of 1/2 malate of Compound (I), and the crystal of the acetate of Compound (I),which were produced according to the methods described in Example 4, Example 5, Comparative Example 1, and Comparative Example 2, were tested in terms of preservation stability under the conditions of Test Example 2.

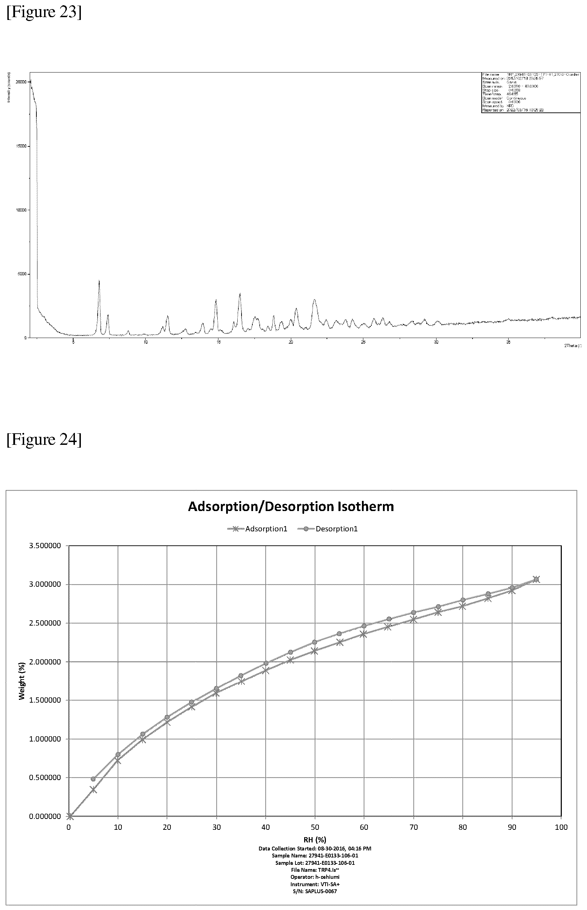

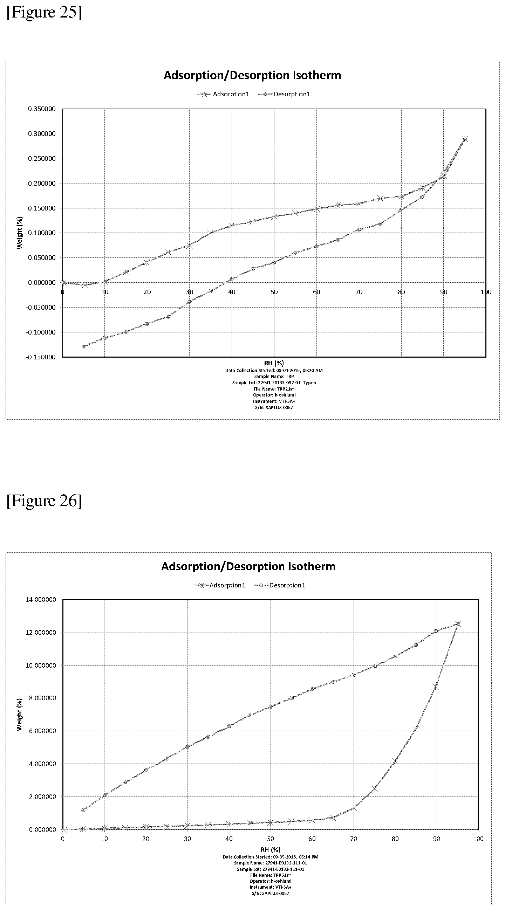

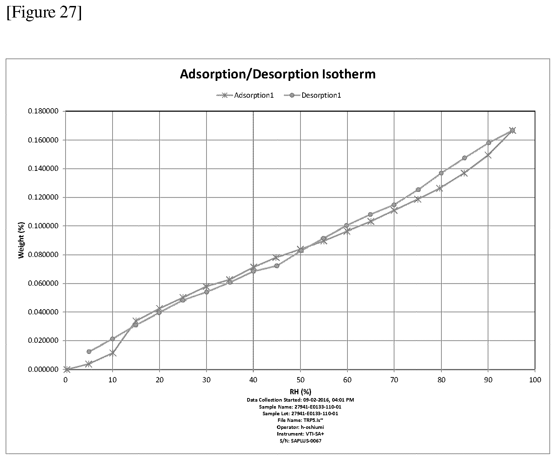

The results are shown in Figure 24, Figure 25, Figure 26, and Figure 27. The crystal of the maleate was confirmed to exhibit low hygroscopicity, and the crystal of the hydrochloride was confirmed to exhibit lower hygroscopicity than the crystal of the 1/2 malate.

## Claims

1. A type I crystal of a free base of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which has peaks at, at least, 2 diffraction angles (2θ±0.2°) selected from the following (a), and at, at least, 2 diffraction angles (2θ±0.2°) selected from the following (b), in a powder X-ray diffraction spectrum (CuKα):
(a) 6.2°, 9.3°, 9.7°, 11.1°, 15.4° and 25.1°; and
(b) 6.7°, 8.3°, 8.7°, 13.0°, 13.7°, 16.3°, 16.9°, 17.7°, 18.7°, 19.4°, 20.3°, 25.9° and 26.5°.

2. The type I crystal of the free base according to claim 1, which has peaks at, at least, 3 diffraction angles (2θ±0.2°) selected from the following (a), and at, at least, 4 diffraction angles (2θ±0.2°) selected from the following (b), in a powder X-ray diffraction spectrum (CuKα):
(a) 6.2°, 9.3°, 9.7°, 11.1°, 15.4° and 25.1°; and
(b) 6.7°, 8.3°, 8.7°, 13.0°, 13.7°, 16.3°, 16.9°, 17.7°, 18.7°, 19.4°, 20.3°, 25.9° and 26.5°.

3. The type I crystal of the free base according to claim 1, which has peaks at the following diffraction angles (2θ±0.2°): 6.2°, 6.7°, 8.3°, 8.7°, 9.3°, 9.7°, 11.1°, 13.0°, 13.7°, 15.4°, 16.3°, 16.9°, 17.7°, 18.7°, 19.4°, 20.3°, 25.1°, 25.9°, and 26.5°, in a powder X-ray diffraction spectrum (CuKα).

4. The type I crystal of the free base according to claim 1, which is a crystal having a powder X-ray diffraction spectrum substantially identical to the powder X-ray diffraction spectrum shown in Figure 1.

5. The type I crystal of the free base according to claim 1, wherein the peak temperature in thermogravimetry-differential thermal analysis (TG-DTA) has an endothermic peak of around 235°C.

6. A pharmaceutical composition, comprising the type I crystal of the free base according to any one of claims 1 to 5.

7. A method for producing the type I crystal of the free base according to claim 1, comprising a step of dissolving 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one in a solvent 1, and a step of adding a solvent 2 to the resulting solution to obtain the 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one.

8. The method according to claim 7, wherein the solvent 1 is dimethyl sulfoxide, and the solvent 2 is water, or the solvent 1 is tetrahydrofuran and the solvent 2 is normal heptane, ethyl acetate, or 2-propanol.

9. The method according to claim 7, wherein the solvent 1 is dimethyl sulfoxide and the solvent 2 is water.

10. The type I crystal of the free base according to claim 1, which is obtained via crystallization by performing a step of dissolving 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one in dimethyl sulfoxide, and a step of adding water to the resulting solution to obtain the 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one.

11. A type II crystal of a free base of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which has peaks at, at least, 2 diffraction angles (2θ±0.2°) selected from the following (a), and at, at least, 2 diffraction angles (2θ±0.2°) selected from the following (b), in a powder X-ray diffraction spectrum (CuKα):
(a) 10.4°, 22.9° and 27.9°; and
(b) 6.7°, 7.5°, 12.7°, 13.4°, 14.6°, 16.5°, 17.9°, 18.3°, 19.8°, 20.6°, 21.8° and 26.5°.

12. The type II crystal of the free base according to claim 11, which has peaks at, at least, 3 diffraction angles (2θ±0.2°) selected from the following (a), and at, at least, 4 diffraction angles (2θ±0.2°) selected from the following (b), in a powder X-ray diffraction spectrum (CuKα):
(a) 10.4°, 22.9° and 27.9°; and
(b) 6.7°, 7.5°, 12.7°, 13.4°, 14.6°, 16.5°, 17.9°, 18.3°, 19.8°, 20.6°, 21.8° and 26.5°.

13. The type II crystal of the free base according to claim 11, which has the following diffraction angles (2θ±0.2°): 6.7°, 7.5°, 10.4°, 12.7°, 13.4°, 14.6°, 16.5°, 17.9°, 18.3°, 19.8°, 20.6°, 21.8°, 22.9°, 26.5° and 27.9°, in a powder X-ray diffraction spectrum (CuKα).

14. The type II crystal of the free base according to claim 11, which is a crystal having a powder X-ray diffraction spectrum substantially identical to the powder X-ray diffraction spectrum shown in Figure 3.

15. The type II crystal of the free base according to claim 11, wherein the peak temperature in thermogravimetry-differential thermal analysis (TG-DTA) has an endothermic peak of around 234°C.

16. A pharmaceutical composition, comprising the type II crystal of the free base according to any one of claims 11 to 15.

17. A type III crystal of a free base of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which has peaks at, at least, 2 diffraction angles (2θ±0.2°) selected from the following (a), and at, at least, 2 diffraction angles (2θ±0.2°) selected from the following (b), in a powder X-ray diffraction spectrum (CuKα):
(a) 11.7°, 23.4° and 24.2°; and
(b) 6.8°, 7.5°, 8.8°, 12.9°, 13.9°, 14.9°, 16.7°, 17.8°, 18.8°, 19.7°, 20.7°, 22.0°, 26.2° and 26.7°.

18. The type III crystal of the free base according to claim 17, which has peaks at, at least, 3 diffraction angles (2θ±0.2°), and at, at least, 4 diffraction angles (2θ±0.2°) selected from the following (b), in a powder X-ray diffraction spectrum (CuKα):
(a) 11.7°, 23.4° and 24.2°; and
(b) 6.8°, 7.5°, 8.8°, 12.9°, 13.9°, 14.9°, 16.7°, 17.8°, 18.8°, 19.7°, 20.7°, 22.0°, 26.2° and 26.7°.

19. The type III crystal of the free base according to claim 17, which has peaks at the following diffraction angles (2θ±0.2°): 6.8°, 7.5°, 8.8°, 11.7°, 12.9°, 13.9°, 14.9°, 16.7°, 17.8°, 18.8°, 19.7°, 20.7°, 22.0°, 23.4°, 24.2°, 26.2° and 26.7°, in a powder X-ray diffraction spectrum (CuKα).

20. The type III crystal of the free base according to claim 17, which is a crystal having a powder X-ray diffraction spectrum substantially identical to the powder X-ray diffraction spectrum shown in Figure 5.

21. The type III crystal of the free base according to claim 17, wherein the peak temperature in thermogravimetry-differential thermal analysis (TG-DTA) has an endothermic peak of around 234°C.

22. A pharmaceutical composition, comprising the type III crystal of the free base according to any one of claims 17 to 21.

23. A crystal of a hydrochloride of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which has peaks at, at least, 3 diffraction angles selected from the following diffraction angles (2θ±0.2°): 6.7°, 7.4°, 11.2', 12.6°, 14.1°, 16.6°, 20.0°, 21.1°, 22.0°, 22.6°, 23.2°, 24.5°, 26.4°, 28.0°, 30.0° and 31.6°, in a powder X-ray diffraction spectrum (CuKα).

24. The crystal of the hydrochloride according to claim 23, which has peaks at the following diffraction angles (2θ±0.2°): 6.7°, 7.4°, 11.2', 12.6°, 14.1°, 16.6°, 20.0°, 21.1°, 22.0°, 22.6°, 23.2°, 24.5°, 26.4°, 28.0°, 30.0° and 31.6°, in a powder X-ray diffraction spectrum (CuKα).

25. The crystal of the hydrochloride according to claim 23, which is a crystal having a powder X-ray diffraction spectrum substantially identical to the powder X-ray diffraction spectrum shown in Figure 7.

26. The crystal of the hydrochloride according to claim 23, wherein the peak temperature in thermogravimetry-differential thermal analysis (TG-DTA) has an endothermic peak of around 241°C.

27. A pharmaceutical composition, comprising the crystal of the hydrochloride according to any one of claims 23 to 26.

28. A crystal of a maleate of 4-(4-(3-((2-(tert-butylamino)ethyl)amino)-6-(5-(trifluoromethyl)-1,3,4-oxadiazol-2-yl)pyridin-2-yl)piperidin-1-yl)-5,5-dimethyl-5H-pyrrolo[2,3-d]pyrimidin-6(7H)-one, which has peaks at, at least, 3 diffraction angles selected from the following diffraction angles (2θ±0.2°): 6.5°, 9.8°, 12.1°, 13.0°, 13.9°, 14.7°, 15.2°, 15.9°, 17.7°, 18.4°, 19.0°, 20.1°, 20.9°, 21.5°, 22.9°, 23.4°, 25.3°, 28.0°, 29.7° and 30.8°, in a powder X-ray diffraction spectrum (CuKα).

29. The crystal of the maleate according to claim 28, which has peaks at the following diffraction angles (2θ±0.2°): 6.5°, 9.8°, 12.1°, 13.0°, 13.9°, 14.7°, 15.2°, 15.9°, 17.7°, 18.4°, 19.0°, 20.1°, 20.9°, 21.5°, 22.9°, 23.4°, 25.3°, 28.0°, 29.7° and 30.8°, in a powder X-ray diffraction spectrum (CuKα).

30. The crystal of the maleate according to claim 28, which is a crystal having a powder X-ray diffraction spectrum substantially identical to the powder X-ray diffraction spectrum shown in Figure 9.

31. The crystal of the maleate according to claim 28, wherein the peak temperature in thermogravimetry-differential thermal analysis (TG-DTA) has an endothermic peak of around 245°C.

32. A pharmaceutical composition, comprising the crystal of the maleate according to any one of claims 28 to 31.
